(19) Europäisches Patentamt
European Patent Office
Office européen des brevets

(11) **EP 2 098 163 A1**

(12) **EUROPEAN PATENT APPLICATION**

(43) Date of publication:
**09.09.2009 Bulletin 2009/37**

(51) Int Cl.:
***A61B 5/029*** (2006.01)    ***A61B 5/083*** (2006.01)

(21) Application number: **09154280.3**

(22) Date of filing: **04.03.2009**

| | |
|---|---|
| (84) Designated Contracting States:<br>**AT BE BG CH CY CZ DE DK EE ES FI FR GB GR HR HU IE IS IT LI LT LU LV MC MK MT NL NO PL PT RO SE SI SK TR**<br>Designated Extension States:<br>**AL BA RS** | (72) Inventors:<br>• **Chang, Keun-Shik**<br>  **305-701, Daejeon (KR)**<br>• **Bae, Hwang**<br>  **Gyeongsangbuk-do 790-751 (KR)** |
| (30) Priority: **04.03.2008 KR 20080020254** | (74) Representative: **Gassner, Wolfgang**<br>**Dr. Gassner & Partner**<br>**Patentanwälte**<br>**Marie-Curie-Strasse 1**<br>**91052 Erlangen (DE)** |
| (71) Applicant: **Korea Advanced Institute of Science and Technology**<br>**Daejeon, 305-701 (KR)** | |

(54) **Method and display apparatus for non-invasively determining pulmonary characteristics by measuring breath gas and blood gas**

(57)    Disclosed are a method for non-invasively determining pulmonary characteristics by measuring breath gas and blood gas and a display apparatus for the same. More particularly, the present invention provides a method for non-invasively determining pulmonary characteristics capable of estimating major physiological characteristics such as respiratory characteristics of lungs-pulmonary circulation system, cardiac functional characteristics, structural characteristics of lungs, etc. by applying primary measurement parameters obtained from ventilation gas and blood during breathing; and a display apparatus useful for the same.

FIG. 5

A: Cardiac output measuring unit
B: ECG measuring unit

EP 2 098 163 A1

## Description

BACKGROUND OF THE INVENTION

This application claims priority to Korean Patent Application No. 2008-0020254, filed on March 04, 2008, in the Korean Intellectual Property Office, the entire contents of which are hereby incorporated by reference.

1. Field of the Invention

The present invention relates to method and display apparatus for non-invasively determining pulmonary characteristics by measuring breath gas and blood gas, more particularly, to a method for non-invasively determining pulmonary characteristics capable of estimating major physiological characteristics such as respiratory characteristics of lungs-pulmonary circulation system, cardiac functional characteristics, structural characteristics of lungs, etc. by applying primary measurement parameters obtained from ventilation gas and blood during breathing; and a display apparatus useful for the same.

2. Description of the Related Art

In general, weight of heart of an adult ranges from 350 to 400g, while length and width thereof are in the range of 12 to 15cm and about 9cm, respectively. Resting heart rate per minute ranges from 60 to 70 and the mean heart beats is about 100,000 for one day and about 2.6 billion for a lifetime in case of a person who lives to 70 years of age. Blood amount circulated in a body is about 5 liters, cardiac output per beat ranges 60 to 70cc while cardiac output per minute ranges from 3.5 to 5.0 liters. Also, a single blood circulation after flowing out of the heart takes about 40 to 50 seconds. Mechanical energy output of the heart per hour is about 6,000cal, which, if totaled for a period of 70 years is approximately equivalent to an amount required to roll a rock weighing 30 tons to the top of Mount Everest.

In a heart, two pumps called right and left ventricles play roles to output blood by pulmonary circulation and systemic circulation, respectively. The heart has an aorta, pulmonary artery, coronary artery, artery, arteriole, peripheral capillary, venules, vein, pulmonary vein, inferior vena cava, superior vena cava and the like and total length thereof is about 96,000km, this is approximately equal to a distance that reaches two and a half times around the earth at the equator.

The heart can spontaneously run by cell aggregation called sinus nodes to collect and discharge electricity at a constant interval of about 0.8 seconds through smooth and rapid pumping of ion channels and Purkinje fibers to appropriately distribute the electricity to muscles near the heart so as to excite, contract and/or relax the muscles with control thereof, in spite of no external power supply.

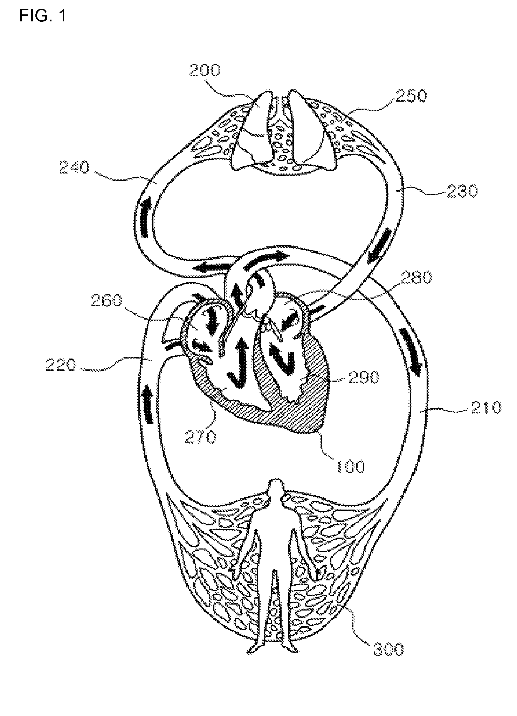

As shown in Fig. 1, the order of blood circulation in the heart and the lungs of a human being is: right ventricle 270 → (pulmonary artery plate) pulmonary artery 240 → pulmonary capillary 250 → pulmonary vein 230 → left atrium 280 → (mitral valve) left ventricle 290 → (aorta plate) aorta 210 → artery → systemic capillary 300 → vein → vena cava 220 → right atrium 260 → (tricuspid valve) right ventricle 270 and the blood circulation is repetitively performed by this route.

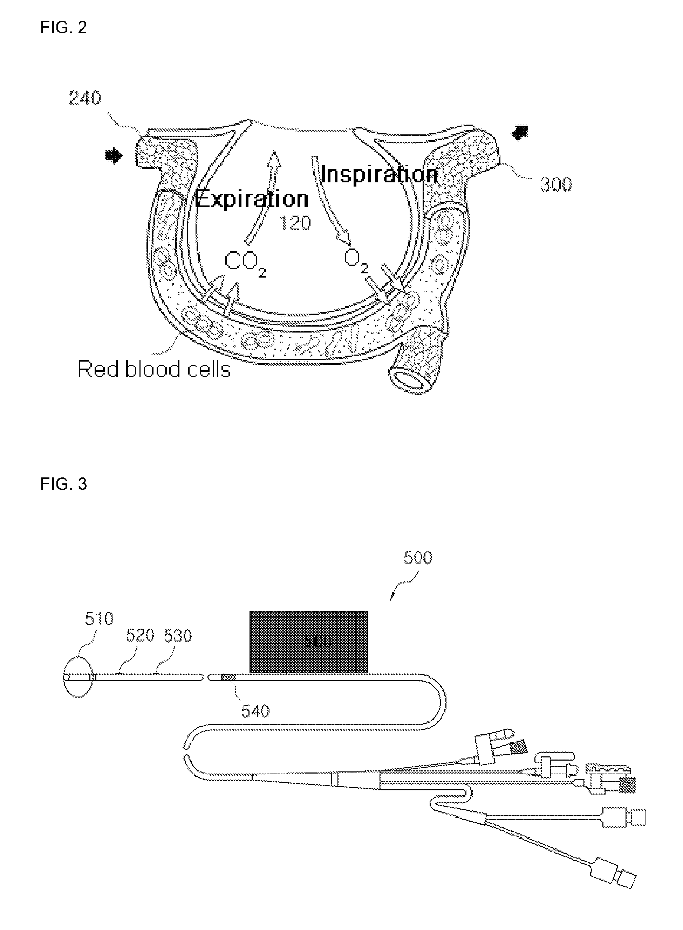

Especially, pulmonary circulation is also called lesser circulation and it means that blood out of the right ventricle 270 reaches to right and left lungs 200 through the pulmonary artery 240, is distributed to a number of capillaries with diameter of about several micrometers around alveoli 120, and returns to the left atrium 280 through the pulmonary vein 230 after very rapid oxygen and carbon dioxide exchange between breathing air and blood through thin alveoli membranes in the capillaries, as shown in Fig. 2. Accordingly, venous blood with concentrated carbon dioxide due to cellular metabolism flows in the pulmonary artery 240 while the pulmonary vein contains oxygen concentrated arterial blood formed by gas exchange.

Such breathing air and blood from the pulmonary circulation are very significant to remove carbon dioxide as waste material and intake oxygen required for metabolism in order to continuously maintain life and become a source of important information useful for medically determining respiratory functions, metabolism, cardiac functions, pulmonary functions, recovery degree and the like in patients with respiratory diseases, especially, serious cases by clinical pathologists.

Furthermore, detailed information related to cardiopulmonary capacities is useful for other applications including, for example: recovery of patients after operation; treating respiratory distress syndrome and solving ventilation problems often occurred in closed spaces such as subway, submarine and/or elevator, in which passengers are in close proximity for longer period of time to increase amount of carbon dioxide; sports medicine in relation to respiratory physiology such as treating high altitude respiratory syndrome of climbers and/or residents caused by thin air or low atmosphere; evaluation of cardiopulmonary functions in patients with damage of respiratory tract or lung cells caused by smoke inhalation due to accidents such as those involving fire; and/or determination of variation in cardiopulmonary capacities of participants in leisure sports such as health running or exercise in health clubs in order to predict health index and/or development of diseases.

However, the direct determination of necessary information obtained from patients by a specialist using an invasive method may cause pain and danger in patients and, occasionally, cannot be achieved. Therefore, it is particularly preferable that parameters of pulmonary characteristics required for clinical pathologists and patients themselves are predicted and offered as a mathematical and physiological model in real time based on information related to shot partial pressure and flow rate of breathing gas, which are determined non-invasively ex vivo, and/or primary information of arterial blood, so as to non-invasively determine the information in real time.

As the first method for determining total amount of blood to be supplied from the heart to the lungs (cardiac output), Korean Patent Application No. 1987-0002027, entitled "catheter for determining cardiac output and catheter for determining blood flow rate", disclosed a direct determination using a catheter illustrated in Fig. 3. The catheter 500 comprises: an opening 510 to discharge liquid in order to determine cardiac output by means of thermodilution; a temperature detection device 530 to determine blood diluted in the liquid including a thermistor 520 as a temperature detection element arranged at a distance apart from the opening 510; and a flow rate signal detection device 540 equipped near the thermistor 520 to determine flow rate signals of the above blood. Such flow rate signal detection device 540 further comprises a self-heating thermistor 520 and the signals for blood flow rate relate to thermal equilibrium temperature detected by the thermistor 520.

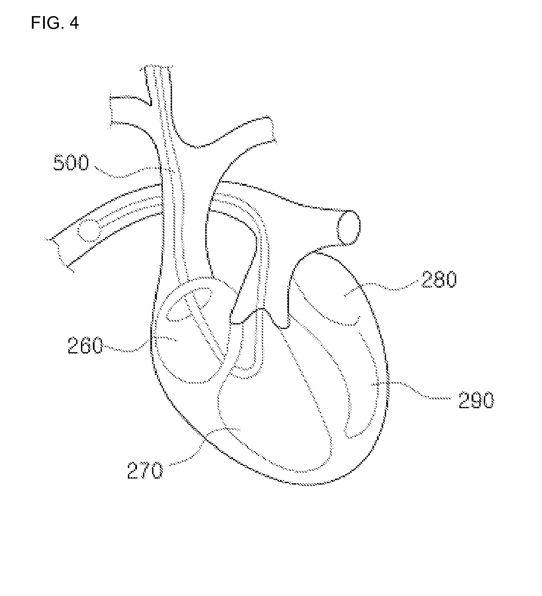

The catheter using method is to estimate cardiac output by comprising: inserting a catheter for pulmonary artery into a jugular vein, femoral vein or femoral stem vein, passing it through superior or inferior vein, right atrium 260 and right ventricle 270, then entering it into pulmonary artery; injecting liquid with higher or lower temperature than blood into the right atrium 260; and detecting temperature of the liquid diffused and diluted in the right atrium 260 and the right ventricle 270 by means of the thermistor positioned in the pulmonary artery, as shown in Fig. 4. This method is an invasive method belonging to thermodilution techniques.

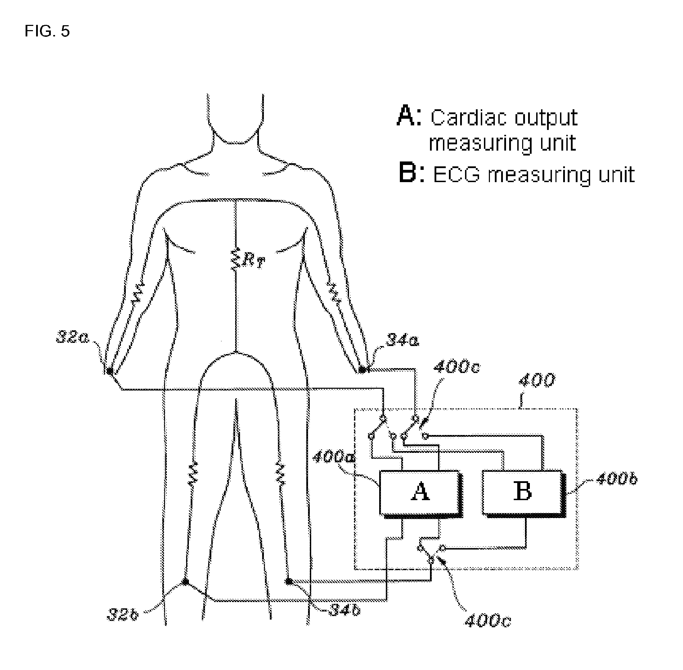

As the second method, Korean Patent Application No. 10-1999-0000417, entitled "Method of attaching electrodes for monitoring ECG and cardiac outputs and apparatus of using the same", disclosed a method for analysis of electrode signals by attaching multiple electrodes on hands (or feet) or arms (or legs) to collect electric signals and analyzing the signals to evaluate cardiac outputs, as shown in Fig. 5.

Referring to Fig. 5 to describe the above method in details, there is illustrated an apparatus comprising; current electrodes 32a and 32b attached on the right hand (or arm) and the right foot (or leg); voltage electrodes 34a and 34b attached on the left hand (or arm) and the left foot (or leg); a switching device 400c for connecting the electrodes 32a and 32b, 34a and 34b to either of a cardiac output measuring unit 400a or electrocardiogram (ECG) measuring unit 400b dependent on control signals; a device for determining cardiac output by applying high frequency current to the current electrodes 32a and 32b connected via the switching device 400c and measuring voltages from the voltage electrodes 34a and 34b; a device for determining ECG by receiving difference signals from the voltage electrodes 34a and 34b via the switching device 400c and measuring ECG based on the difference signals; and a control device that offers control signals to the switching device 400c and displays measured values of the cardiac output determining device and the ECG determining device on a display.

The third one is a non-invasive evaluation of cardiac output by measuring expiration gas, so-called NICO (Non Invasive Cardiac Output) and can be exemplified by "Partial $CO_2$ rebreathing method" developed by Novametrix Medical Systems. This method comprises measuring unitial pressure of $CO_2$ in the expiration gas and obtaining solutions of Fick's equation for $CO_2$ with the measured partial pressure to evaluate cardiac outputs. The above method needs simple input parameters without requiring alternative information for $O_2$ diffusion, however, has a deficit of poor prediction accuracy.

The fourth one is a method for determining $O_2$ partial pressure in mixed venous blood by applying detailed input parameters such as measured cardiac output, $O_2$ uptake and/or $O_2$ partial pressure in arterial blood and solving Fick's equation for $O_2$, as disclosed in Korean Patent Laid-Open No. 1999-22493.

The first method is a severe invasive method and even though it has high accuracy it may possibly cause pain, complications and/or infection in patients at the time of performing the method. The second method has a trouble in mounting electrodes on desired sites of a human body. The third method has an advantage in that it has a simple solving process by only applying measured value of $CO_2$ partial pressure to Fick's equation. However, it is well known that this method exhibits poor reliability unless the cardiac output reaches about 6 liters/min, since the equation has insufficient numerical factors and lack of information for $O_2$ combined with red blood cells in blood to cause reduction in amount of information to be predicted and poor accuracy.

Likewise, since the fourth method adopts a limited numerical formula such as Fick's law for $O_2$ and uses cardiac output information obtained from a transducer (piezoelectric sensor) for detection of arterial wave, the method has disadvantages such as uncomfortable use, poor accuracy and/or restricted information due to prediction of only $O_2$ partial pressure except $CO_2$ partial pressure in mixed venous blood. In contrast, the present invention applies more complex numerical formulae including, for example, mass balance equation to preserve all of masses for $O_2$ and $CO_2$, shunt rate equation, respiration quotient ratio equation, ventilation-perfusion ratio equation, etc., and can speedily calculate shunt rate of lungs, dead space rate, shot partial pressure information of peripheral capillaries and the like as well as cardiac output and shot partial pressure information of mixed veins on the basis of classification of respiration models and analysis methods, so that the present invention can non-invasively offer useful and accurate medical information in real time, thus, is clearly different from conventionally known arts described above in view of technical idea.

A human lung has some shunt and physiological dead space even in a healthy person and, especially, a patient with respiratory disease has severe shunt and physiological dead space causing respiratory function to be significantly reduced. Therefore, pulmonary characteristics predicted by any methods carried out without considering shunt and physiological dead space are obtained for an ideal condition of a lung and are certainly considered to have errors and/or differences from clinically measured values for real life patients. Although respiratory problems were defined and analyzed in consideration of shunt and dead space, the analyzed results may involve significant error based on numerical formulae relating to respiratory equations and accuracy of solutions for the numerical formulae.

The present invention provides an improved method to determine shunt and physiological dead space and, at the same time, systematically determine different respiratory parameters including, for example, partial gas pressure of pulmonary alveolus, alveolus ventilation, flow rate, cardiac output, respiration quotient ratio and so on by applying a variety of more complex numerical formulae such as mass balance equation for $O_2$, mass balance equation for $CO_2$, shunt rate equation for $O_2$, shunt rate equation for $CO_2$, respiration quotient ratio equation for ventilation, respiration quotient ratio equation for blood, ventilation-perfusion ratio equation for $O_2$, ventilation-perfusion ratio equation for $CO_2$, etc. through classification of three kinds of respiration models.

A gas exchanging process in a lung means that blood simultaneously releases $CO_2$ and receives $O_2$ by diffusion, and must have a connection for reversing increase and decrease of partial pressures both of the gases. In general, shot partial pressure A* of the pulmonary alveolar gas is substantially equal to shot partial pressure C* of peripheral capillaries (that is, A*=C*) through equilibrium at the end of expiration. If there is physiological dead space in a lung, non-functional air not used in gas exchange is mixed in the lung so that the gas at the end of expiration has lower $CO_2$ partial pressure and higher $O_2$ partial pressure compared to functional air after completion of the gas exchange in the pulmonary alveolus. If there is shunt in the lung, non-functional blood not used in gas exchange is mixed in the lung so that the arterial blood has lower $O_2$ partial pressure and higher $CO_2$ partial pressure compared to functional blood after completion of the gas exchange in the capillary.

Accordingly, determination of partial gas pressure for either the gas at the end of expiration or the arterial blood cannot be connected by prediction of partial gas pressure for gas in pulmonary alveolus and/or peripheral capillaries and collection of pulmonary alveolar gas or blood in capillaries itself is very difficult, so that it is substantially impossible to find out shot partial gas pressure of pulmonary alveolar gas or blood in capillaries through direct measurement. Consequently, with considering shunt or physiological dead space, it is very important to predict physiological characteristics of cardiopulmonary organs of a human body and there is a strong requirement for more advanced ideas and techniques to provide non-invasive and systematically rapid prediction means and/or instruments, compared to conventional methods known in the art.

SUMMARY OF THE INVENTION

Accordingly, the present invention has been proposed to solve problems such as difficulty, danger, side effects, uncertainty, delayed response and/or restriction in conventional techniques described above and an object of the present invention is to provide a method for computer analysis of numerical formulae systems such as mass balance equations for $O_2$ and $CO_2$, comprising: measuring flow rates of inspiration and/or expiration air in ventilation gas during breathing, and shot partial pressures thereof; measuring shot partial pressure of arterial blood and applying as an input parameter; and using the input parameter to analyze the numerical formulae system. And the present invention further provides a method for non-invasively determining pulmonary characteristics by applying the solutions obtained from the computer analysis to pre-determine a provisional data region for mixed vein, so that it can accurately predict shot partial pressure of mixed venous blood, shot partial pressure of peripheral capillary blood, shot partial pressure of pulmonary alveolar gas, cardiac output, shunt rate and physiological dead space rate in respiratory organs and, in addition, a display apparatus useful for the same. In order to accomplish the above described objects, the present invention provides a method for determining pulmonary characteristics, comprising: (a) inputting respiratory input parameters including additional blood information, gas boundary value, additional gas information, inspiration flow rate, etc. into an automatic operation device; (b) inputting an initial value of shot concentration of mixed vein V into the automatic operation device; (c) inputting an initial value of dead space rate X into the automatic operation device; (d) inputting an initial value of $O_2$ partial pressure of pulmonary alveolar gas A1 based on the initial value of dead space rate X as respiratory input parameters into the automatic operation device; (e) applying the respiratory input parameters and the inputted initial values to an operational routine provided in the automatic operation device, so as to obtain a solution from equations for respiratory gas including mass balance equations for $O_2$, $CO_2$ and/or $N_2$ as well as Fick's equation; (f) calculating an estimated value of $CO_2$ partial pressure of pulmonary alveolar gas A2 as a solution obtained from analytical equations for respiratory gas via the operational routine; (g) calculating $O_2$ shunt rate Y1 and $CO_2$ shunt rate Y2 based on the initial value of $O_2$ partial pressure and the estimated $CO_2$ partial pressure of pulmonary alveolar gas A1 and A2; (h) determining pulmonary characteristics related to shot partial pressure of pulmonary alveolar gas A*, when shunt rate requirements are satisfied; (i) repeating steps (b) to (h) with plural shot partial pressures of mixed vein (V*)n at a constant interval as initial values so as to determine plural shot partial pressures of pulmonary alveolar gas (A*)n corresponding to the plural shot partial pressures of mixed vein (V*)n, respectively, as well as pulmonary characteristics related thereto; (j) when a specific shot pressure of pulmonary alveolar gas A** satisfying requirements for respiratory rate is selected from the plural shot partial pressures of pulmonary alveolar gas (A*)n, determining a specific pulmonary characteristic corresponding to the specific shot pressure of pulmonary alveolar gas A**; and (k) calculating cardiac output.

As to step (a) described above, the additional blood information may comprise shot partial pressure of arterial blood a* or measured $O_2$ partial pressure thereof. The gas boundary value may comprise shot partial pressure of inspired gas I* or measured $O_2$ partial pressure thereof. And, the additional gas information may comprise shot partial pressure of gas ET* at the end of expiration or measured $CO_2$ partial pressure thereof. The inspiration flow rate $\dot{V}_I$ means total flow rate of external air received by lungs, while an expiration flow rate VE means total flow rate of air released from the lungs. Such $\dot{V}_I$ may be setup to be the substantially same level as VE.

As to step (h) described above, the shunt rate requirements are to compare and determine whether a difference between the $O_2$ shunt rate Y1 and the $CO_2$ shunt rate Y2 is within a desired range. Additionally, as to step (f) described above, the analytical equations for respiratory gas may comprise mass balance equations for $O_2$, $CO_2$ and/or $N_2$.

As to step (i) described above, the plural shot partial pressures of mixed vein (V*)n correspond to lattice points of a plurality of divided "mixed vein lattices", respectively, each being setup as a blood boundary value and inputted into the automatic operation device. The lattice points of the plural "mixed vein lattices" may have irregular spaces between lattices, comprise multi-grids including larger and smaller grids combined together and, optionally, a primary larger grid capable of being further re-divided into smaller ones. In order to accomplish the above described objects, there is also provided a method for determining pulmonary characteristics, comprising : (a) inputting respiratory input parameters including additional blood information, gas boundary value, additional gas information, inspiration flow rate, etc. into an automatic operation device; (b) inputting an initial value of shot concentration of mixed vein V into the automatic operation device; (c) inputting an initial value of shunt rate Y into the automatic operation device; (d) inputting an initial value of $O_2$ partial pressure of pulmonary alveolar gas A1 based on the initial value of shunt Y as respiratory input parameters into the automatic operation device; (e) applying the respiratory input parameters and the inputted initial values to an operational routine provided in the automatic operation device, so as to obtain a solution from equations for respiratory gas including mass balance equations for $O_2$, $CO_2$ and/or $N_2$ as well as Fick's equation; (f) calculating an estimated value of $CO_2$ partial pressure of pulmonary alveolar gas A2 as a solution obtained from analytical equations for respiratory gas via the operational routine; (g) calculating $O_2$ dead space rate X1 and $CO_2$ dead space rate X2 based on the initial value of $O_2$ partial pressure and the estimated $CO_2$ partial pressure of pulmonary alveolar gas A1 and A2; (h) determining pulmonary characteristics related to shot partial pressure of pulmonary alveolar gas A*, when dead space rate requirements are satisfied; (i) repeating steps (b) to (h) with plural shot partial pressures of mixed vein (V*)n at a constant interval as initial values so as to determine plural shot partial pressures of pulmonary alveolar gas (A*)n corresponding to the plural shot partial pressures of mixed vein (V*)n, respectively, as well as pulmonary characteristics related thereto; (j) when a specific shot pressure of pulmonary alveolar gas A** satisfying requirements for respiratory rate is selected from the plural shot partial pressures of pulmonary alveolar gas (A*)n, determining a specific pulmonary characteristic corresponding to the specific shot pressure of pulmonary alveolar gas A**; and (k) calculating cardiac output.

As to step (h) described above, the dead space rate requirements are to compare and determine whether a difference between the $O_2$ dead space rate X1 and the $CO_2$ dead space rate X2 is within a desired range. In order to accomplish the above described object, there is also provided a method for determining pulmonary characteristics, comprising: (a) inputting respiratory input parameters including additional blood information, gas boundary value, additional gas information, inspiration flow rate, etc. into an automatic operation device; (b) inputting an initial value of shot concentration of mixed vein V into the automatic operation device; (c) inputting an initial value of dead space rate X into the automatic operation device; (d) inputting an initial value of $CO_2$ partial pressure of pulmonary alveolar gas A2 based on the initial value of dead space rate X as respiratory input parameters into the automatic operation device; (e) applying the respiratory input parameters and the inputted initial values to an operational routine provided in the automatic operation device, so as to obtain a solution from equations for respiratory gas including mass balance equations for $O_2$, $CO_2$ and/or $N_2$ as well as Fick's equation; (f) calculating an estimated value of $O_2$ partial pressure of pulmonary alveolar gas A1 as a solution obtained from analytical equations for respiratory gas via the operational routine; (g) calculating $O_2$ shunt rate Y1 and $CO_2$ shunt rate Y2 based on the initial value of $CO_2$ partial pressure and the estimated $O_2$ partial pressure of pulmonary alveolar gas A2 and A1; (h) determining pulmonary characteristics related to shot partial pressure of pulmonary alveolar gas A*, when shunt rate requirements are satisfied; (i) repeating steps (b) to (h) with plural shot partial pressures of mixed vein (V*)n at a constant interval as initial values so as to determine plural shot partial pressures of pulmonary alveolar gas (A*)n corresponding to the plural shot partial pressures of mixed vein (V*)n, respectively, as well as pulmonary characteristics related thereto; (j) when a specific shot pressure of pulmonary alveolar gas A** satisfying requirements for respiratory rate is selected from the plural shot partial pressures of pulmonary alveolar gas (A*)n, determining a specific pulmonary characteristic corresponding to the specific shot pressure of pulmonary alveolar gas A**; and (k) calculating cardiac output.

As to step (h) described above, the shunt rate requirements are to compare and determine whether a difference between the $O_2$ shunt rate Y1 and the $CO_2$ shunt rate Y2 is within a desired range. In order to accomplish the above described object, there is also provided a method for determining pulmonary characteristics, comprising: (a) inputting respiratory input parameters including additional blood information, gas boundary value, additional gas information, inspiration flow rate, etc. into an automatic operation device; (b) inputting an initial value of shot concentration of mixed vein V into the automatic operation device; (c) inputting an initial value of shunt rate Y into the automatic operation device; (d) inputting an initial value of $CO_2$ partial pressure of pulmonary alveolar gas A2 based on the initial value of shunt rate Y as respiratory input parameters into the automatic operation device; (e) applying the respiratory input parameters and the inputted initial values to an operational routine provided in the automatic operation device, so as to obtain a solution from equations for respiratory gas including mass balance equations for $O_2$, $CO_2$ and/or $N_2$ as well as Fick's equation; (f) calculating an estimated value of $O_2$ partial pressure of pulmonary alveolar gas A1 as a solution obtained from analytical equations for respiratory gas via the operational routine; (g) calculating $O_2$ dead space rate X1 and $CO_2$ dead space rate X2 based on the initial value of $CO_2$ partial pressure and the estimated $O_2$ partial pressure of pulmonary alveolar gas A2 and A1; (h) determining pulmonary characteristics related to shot partial pressure of pulmonary alveolar gas A*, when dead space rate requirements are satisfied; (i) repeating steps (b) to (h) with plural shot partial pressures of mixed vein (V*)n at a constant interval as initial values so as to determine plural shot partial pressures of pulmonary alveolar gas (A*)n corresponding to the plural shot partial pressures of mixed vein (V*)n, respectively, as well as pulmonary characteristics related thereto; (j) when a specific shot pressure of pulmonary alveolar gas A** satisfying requirements for respiratory rate is selected from the plural shot partial pressures of pulmonary alveolar gas (A*)n, determining a specific pulmonary characteristic corresponding to the specific shot pressure of pulmonary alveolar gas A**; and (k) calculating cardiac output.

The dead space rate requirements in step (h) are to compare and determine whether a difference between the $O_2$ dead space rate X1 and the $CO_2$ dead space rate X2 is within a desired range.

As to step (j) described above, the requirements for respiratory rate are to compare and determine whether a difference between a respiratory rate obtained from calculated numerical values and another respiratory rate obtained from the difference between shot partial pressures of inspired gas and gas at the end of expiration is within a desired range. The specific pulmonary characteristic determined in step (j) means any one selected from shot partial pressure of pulmonary alveolar gas A**, shot partial pressure of peripheral capillary blood C**, ventilation-perfusion ratio $(\dot{V}_A/Q)$ **, shunt rate Y** and physiological dead space rate X**.

In step (k), the cardiac output may be calculated using measured amount of inspired air $\dot{V}_I$ or of expired air VE and the physiological dead space rate X**.

As to step (a) described above, the additional blood information only includes shot $O_2$ partial pressure of arterial blood a1*, while repeating steps (a) to (k) may determine shot $CO_2$ partial pressure of arterial blood a2*.

Additionally, in order to accomplish the above object of the present invention, there is provided an apparatus for displaying pulmonary characteristics, comprising an information terminal connected to an automatic operation device to visibly display pulmonary characteristics determined by any one of the methods for determining pulmonary characteristics according to the present invention described above.

Particularly, such an apparatus for displaying pulmonary characteristics has a portable type information terminal capable being wired/wirelessly connected to the automatic operation device, wherein the automatic operation device may comprise computer processors and/or embedded chips provided in a computer.

The method according to the present invention is characterized in that: a measurement device for non-invasively determining pulmonary characteristics is used; three kinds of respiration model for sequentially solving and analyzing complicated problems are adopted to evaluate physiological characteristics such as blood respiration characteristics of lungs-pulmonary circulation system, cardiac functional characteristics, lung functional characteristics and the like; and different computer analysis processes for three respiration models and corresponding operation devices for the same are applied. Therefore, the inventive method is very accurate and effectively used in any case whether shunt and/or physiological dead space exists in lungs or not.

Compared to conventional techniques such as thermodilution that inserts a pulmonary arterial catheter into the pulmonary artery via the right ventricle and the right atrium, the present invention can eliminate pain, infection and/or complications of patients possibly caused by the catheter operation. Moreover, the present invention has no trouble of using electrodes required for receiving electrical bio-signals by attaching or fixing the electrodes to correct sites on hands or arms and legs or feet. The present invention also considers equilibriums by $O_2$ diffusion as well as $CO_2$ diffusion in pulmonary capillary. Therefore, compared to $CO_2$-rebreathing method developed by Novametrics Co. which uses only $CO_2$ data obtained by breathing and is effective in a specific narrow range of cardiac outputs, various respiratory characteristics in lungs-pulmonary circulation system can be predicted or determined in more extended range of cardiac outputs.

BRIEF DESCRIPTION OF THE DRAWINGS

These and other objects, features, aspects, and advantages of the present invention will be more fully described in the following detailed description of preferred embodiments, taken in conjunction with the accompanying drawings. In the drawings:

Fig. 1 is a schematic view illustrating order of blood circulation in heart and lungs of a human body;
Fig. 2 is a schematic view illustrating $O_2$ and $CO_2$ exchange during pulmonary circulation in a human body;
Fig. 3 is a view illustrating a method for determining total amount(cardiac output) of blood supply to lungs using a catheter according to a conventional method;
Fig. 4 is a schematic view illustrating in detail a method for calculating cardiac output using a catheter according to the conventional method shown in Fig. 3;
Fig. 5 is a schematic block diagram illustrating an electrode signal analysis as a conventional method that attaches multiple electrodes on hands or feet to collect electric signals and analyzes the signals to evaluate cardiac outputs;
Fig. 6 is a view illustrating gas exchange in pulmonary alveolus of a human body;
Fig. 7 is a diagram illustrating $O_2$-$CO_2$ partial pressure relation in pulmonary alveolus of a human body;
Fig. 8 is a block diagram illustrating an apparatus for determining respiratory characteristics in lungs-pulmonary circulation system according to an exemplary embodiment of the present invention;
Fig. 9 is a flow chart illustrating a method for determining pulmonary characteristics according to a first type of a third respiration model in the exemplary embodiment of the present invention;
Fig. 10 is a flow chart illustrating a method for determining pulmonary characteristics according to a second type of the third respiration model in the exemplary embodiment of the present invention;
Fig. 11 is a flow chart illustrating a method for determining pulmonary characteristics according to a third type of the third respiration model in the exemplary embodiment of the present invention;
Fig. 12 is a flow chart illustrating a method for determining pulmonary characteristics according to a fourth type of the third respiration model in the exemplary embodiment of the present invention; and
Fig. 13 is a graph illustrating ventilation-perfusion ratio curves according to the exemplary embodiment of the present invention.

DETAILED DESCRIPTION OF THE INVENTION

All terms including technical and scientific terms used herein have generally the same meaning as commonly understood by those skilled in the art, however optionally, are otherwise defined by the present applicant. In this case, the specified terms are described in detail in the disclosure and must be interpreted as having alternative meaning different from that in the context of the relevant art.

Hereinafter, preferred embodiments of the present invention to achieve the purposes described above will be described in detail with reference to the accompanying drawings, which are only given for the purpose of illustration and are not to be construed as limiting the scope of the invention. Therefore, it will be understood by those skilled in the art that various modifications and variations including technical spirits of the present invention may be made therein without departing from the scope of the present invention.

Referring to Fig. 1, blood circulation in a human body is typically classified into two kinds of circulation, that is, systemic circulation and pulmonary circulation. Systemic circulation is to supply $O_2$ to tissues in cell units constructing each of organs in a human body and recover $CO_2$ from the same, while pulmonary circulation is to discharge the recovered $CO_2$ out of the body and receive $O_2$. After the systemic circulation, the blood flows from the right ventricle to pulmonary artery and starts the pulmonary circulation. Deoxygenated mixed venous blood is exchanged with fresh air supplied through respiratory organs in pulmonary alveolus as a peripheral organ of a lung to become arterial blood, then, flows through pulmonary vein to the left ventricle.

However, the air supplied to pulmonary alveolus has dead space in which the gas cannot be exchanged with pulmonary capillary blood, while the blood has a shunt in which the blood cannot be exchanged with pulmonary alveolus air. As a result, the discharged air includes air contained in the dead space and thus may have $O_2$-$CO_2$ composition different from that of the pulmonary alveolus air which was resulted from gas exchange. Similarly, the arterial blood after the pulmonary circulation includes the mixed venous blood contained in the shunt and may also have $O_2$-$CO_2$ composition different from that of the pulmonary peripheral capillary blood which was completely oxygenated.

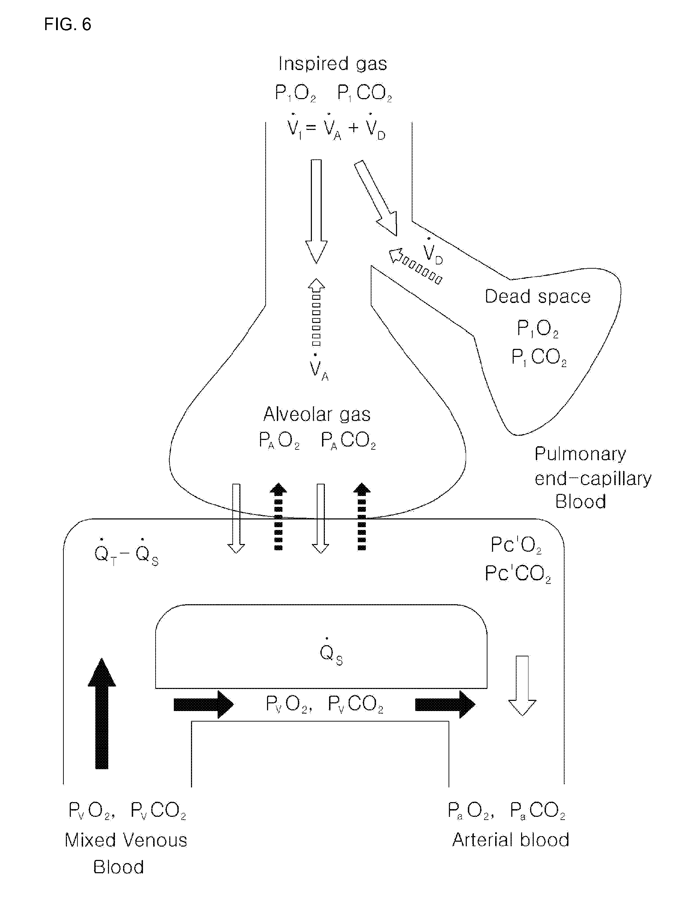

Referring to Fig. 6 for illustrating gas exchange in pulmonary alveolus of the human body, the gas exchange process is primarily defined by a 3-compartment lung model consisting of 1) pulmonary alveolus and pulmonary capillary blood, 2) shunt and 3) dead space (sometimes, the gas exchange process is defined by a 5-compartment lung model since the gas exchange between pulmonary alveolus and pulmonary capillary blood is classified according to high or low ventilation-perfusion ratio ($\dot{V}_A/\dot{Q}$).

Symbols used in the equations have meanings as follows:

$C_{a_{CO2}}$ : $CO_2$ concentration in arterial blood [%]

$C_{a_{O2}}$ : $O_2$ concentration in arterial blood [%]

$C_{C_{CO2}}$ : CO2 concentration in capillary [%]

$C_{C_{O2}}$ :$O_2$ concentration in capillary [%]

$C_{\bar{V}_{CO2}}$ : $CO_2$ concentration in mixed vein [%]

$C_{\bar{V}_{CO2}}$ : $O_2$ concentration in mixed vein [%]

$F_{I_{CO2}}$ : $CO_2$ partial pressure ratio in atmosphere

$F_{I_{N2}}$ : $N_2$ partial pressure ratio in atmosphere

$P_{A_{CO2}}$ : $CO_2$ partial pressure in pulmonary alveolus [mmHg]

$P_{A_{H2O}}$ : water vapor pressure in pulmonary alveolus [mmHg]

$P_{A_{N2}}$ : $N_2$ partial pressure in pulmonary alveolus [mmHg]

$P_{A_{O2}}$ : $O_2$ partial pressure in pulmonary alveolus [mmHg]

$P_B$ : atmospheric pressure [mmHg]

$P_{C_{N2}}$ : $N_2$ partial pressure of capillary [mmHg]

$P_{I_{N2}}$ : $N_2$ partial pressure of air in atmosphere [mmHg]

$P_{I_{O2}}$ : $O_2$ partial pressure of air in atmosphere [mmHg]

$P_{\bar{V}_{N2}}$ : $N_2$ partial pressure of mixed vein [mmHg]

$Q$ : perfusion of capillary [liters/min]

$Q_S$ : shunt flow rate **[liters/min]**

$Q_T$ : cardiac output [liters/min]

$\dot{V}_A$ : flow rate of air gas-exchanged in pulmonary alveolus [liters/min]

$\dot{V}_D$ :flow rate of dead space air [liter/min]

$\dot{V}_T$ : total inspiration or expiration capacity [liters/min]

$\dot{V}_I$ :flow rate of inspiration air [liters/min]

$\dot{V}_{CO2}$ carbon dioxide output into pulmonary alveolus [ml/min]

$\dot{V}_{O2}$ :oxygen uptake into capillary [ml/min]

R :respiratory exchange ratio

$R_B$ : respiratory exchange ratio on blood side

$R_G$ : respirator exchange ratio on gas side

X :dead space rate

Y :shunt rate

$\kappa$ :respiratory quotient

$\lambda$ : constant for blood and air

Some gases ($O_2$, $CO_2$, and $N_2$) supplied to pulmonary alveolus through air inspiration are exchanged with gases in the mixed venous blood flowed from the right ventricle to the pulmonary capillaries through alveolar membrane while mutually preserving masses thereof. Such a relation is mathematically expressed by the following mass balance equations such as Eq.1 to Eq.4:

$$(\dot{V_I}/\dot{Q})P_{I_{O2}} - (\dot{V_A}/\dot{Q})P_{A_{O2}} = \kappa \times (C_{C_{O2}} - C_{\overline{V}_{O2}}) \tag{1}$$

$$(\dot{V_A}/\dot{Q})_2 P_{A_{CO2}} = \kappa(C_{\overline{V}_{CO2}} - C_{C_{CO2}}) \tag{2}$$

$$(\dot{V_I}/\dot{Q})_3 P_{I_{N2}} - (\dot{V_A}/\dot{Q})_3 P_{A_{N2}} = \lambda(P_{C_{N2}} - P_{\overline{V}_{N2}}) \tag{3}$$

$$P_{A_{O2}} + P_{A_{N2}} + P_{A_{CO2}} = P_B + P_{A_{H2O}} \tag{4}$$

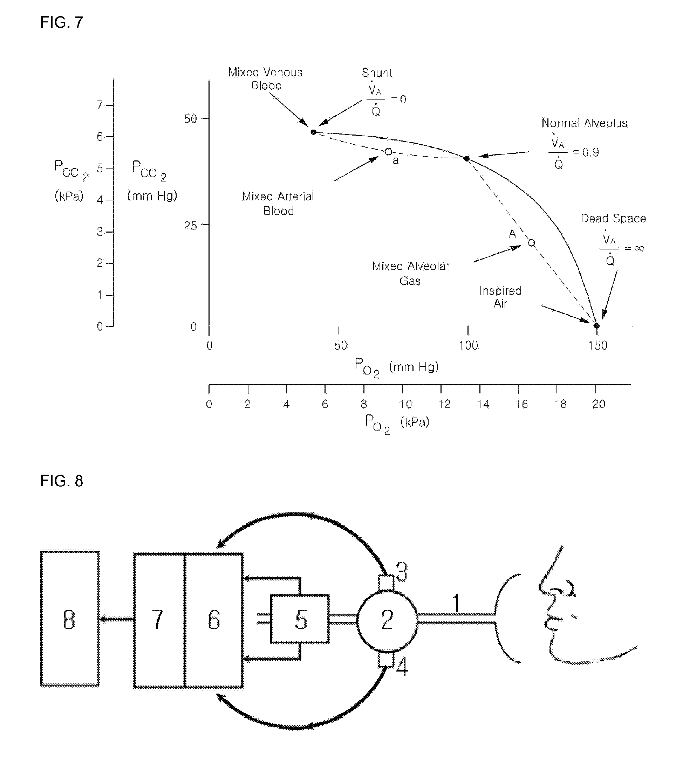

Fig. 7 is a diagram illustrating $O_2$-$CO_2$ partial pressure relation in pulmonary alveolus of a human body. Using $O_2$-$CO_2$ partial pressures of the mixed venous blood and the inspired air as input values to solve the mass balance equations, a $O_2$-$CO_2$ diagram comprising a curve with both end points corresponding to the above input values may be obtained, which substantially exhibits mass preservation of $O_2$-$CO_2$ partial pressures, as shown in Fig. 7.
Any one point on the curve means not only $O_2$-$CO_2$ partial pressures of alveolar gas satisfying the mass balance equations, but also a corresponding ventilation-perfusion ratio ($\dot{V_A}/\dot{Q}$). In general, the ventilation-perfusion ratio has a distribution ranging from 0 to infinity on the $O_2$-$CO_2$ diagram. The left end point is a $O_2$-$CO_2$ partial pressure point of the mixed venous blood as a ventilation point ( ($\dot{V_A}=0(\dot{V_A}/Q=0)$) ), which is substantially the same as a partial pressure of shunt blood. The right end point is a $O_2$-$CO_2$ partial pressure point of the inspired air as a perfusion point ($\dot{Q}=0(\dot{V_A}/\dot{Q}=\infty)$), which is substantially the same as a partial pressure of dead space air.
It is well known that a normal person has the shunt rate of less than 7% and the dead space rate of about 25%. Compared to upper part of a lung, both of ventilation and perfusion increase in a lower part of the lung. Since increase of perfusion is higher than that of the ventilation, the ventilation-perfusion ratio is highest at the uppermost part of the lung and gradually decreases toward the lower part of the lung. Accordingly, the pulmonary end-capillary blood obtained after full oxygenation by gas exchange through alveolar membrane may have oxygen concentration different from that of arterial blood which combines with blood not fully oxygenated due to shunt or heterogeneity of ventilation-perfusion ratio in pulmonary vein, and then, returns to the left atrium.

Likewise, alveolar gas resulted from gas exchange with blood in pulmonary alveolus contains dead space air having the same $O_2$-$CO_2$ partial pressure as the inspired air and is discharged out of the body during expiration, thus, the alveolar gas may have $O_2$-$CO_2$ partial pressure different from that of the expired air. Accordingly, $O_2$-$CO_2$ partial pressure of the alveolar gas must be determined with considering different elements caused by blood circulation of heart as well as respiratory function of lungs.

A difference between $O_2$-$CO_2$ concentrations of mixed venous blood ($\bar{C}_V$) and arterial blood ($C_a$) directly correlates with $O_2$ consumption ($\dot{V}_{O2}$) and $CO_2$ production ($\dot{V}_{CO2}$) in tissues in view of metabolism and may be numerically defined by Fick's principle which is represented by the following equations Eq. 5 and Eq. 6:

$$\dot{V}_{O2} = \dot{Q}\left(C_{a_{O2}} - C_{\bar{V}_{O2}}\right)$$

$$(5)$$

$$\dot{V}_{CO2} = \dot{Q}\left(C_{\bar{V}_{CO2}} - C_{a_{CO2}}\right)$$

$$(6)$$

In a normal condition with stable respiration, respiratory quotient ($RQ = \dot{V}_{CO2}/\dot{V}_{O2}$) meaning a ratio of $O_2$ consumption to $CO_2$ production in view of metabolism, is substantially identical to respiratory exchange ratio R derived from mass balance equations to express a ratio of $O_2$ uptake to $CO_2$ elimination, which are conducted between pulmonary alveolus and pulmonary capillary blood. If respiratory volume increases or decreases during breathing, the respiratory exchange ratio in pulmonary alveolus alters faster than that during metabolism. Therefore, the above two parameters $RQ$ and R are not always the same as each other. The respiratory exchange ratio in pulmonary alveolus may be expressed by the following equations Eq. 7 and Eq. 8:

$$R_B \equiv \frac{C_{\bar{V}_{CO2}} - C_{C_{CO2}}}{C_{C_{O2}} - C_{\bar{V}_{O2}}}$$

$$(7)$$

$$R_G \equiv \frac{P_{A_{CO2}}\left(1 - F_{I_{O2}}\right)}{P_{I_{O2}} - F_{I_{O2}} P_{A_{CO2}} - P_{A_{O2}}}$$

$$(8)$$

However, two parameters described above may provide detailed information related to respiratory gas/blood in a normal condition. Firstly, using $RQ$ and $R_B$ may define $O_2$-$CO_2$ concentrations in mixed venous blood, arterial blood and pulmonary end-capillary blood by a numerical relation. $O_2$-$CO_2$ concentration distribution outlined by three points satisfying the above relation may exist on a line segment. $R_G$ is an equation expressing $O_2$-$CO_2$ partial pressures of inspired air and alveolar gas. If $O_2$-$CO_2$ partial pressure of the alveolar gas is the same as that of the pulmonary end-capillary blood, $O_2$-$CO_2$ partial pressure of the alveolar gas may be obtained from $R_G$ and $R_B$. In a clinical case, $O_2$ partial pressure in pulmonary alveolus $P_{A_{O2}}$ may be calculated by the following equation Eq. 11 which is alveolar ventilation equation for $O_2$ and $CO_2$ derived from Eq. 9 and Eq. 10. However, the value calculated by Eq. 11 is more uncertain than that calculated by the following equation Eq. 12 derived from Eq. 8. Moreover, Eq. 9 and Eq. 10 are primarily based on a presumption that $CO_2$ partial pressure in the alveolar gas $P_{A_{CO2}}$ is substantially the same as that in the arterial blood $P_{a_{CO2}}$. A difference between these $CO_2$ partial pressures is typically small and ranges from 1 to 3 mmHg, however, may increase for a patient suffering from respiratory distress. Therefore, occasionally, $O_2$ partial

pressure in pulmonary alveolus obtained from Eq. 11 may not be a true value.

$$P_{I_{O2}} - P_{A_{O2}} = \frac{\dot{V}_{O2}}{\dot{V}_A}(P_B - P_{A_{H2O}})$$

$$(9)$$

$$P_{A_{CO2}} = \frac{\dot{V}_{CO2}}{\dot{V}_A}(P_B - P_{A_{H2O}})$$

$$(10)$$

$$P_{A_{O2}} = P_{I_{O2}} - \frac{P_{a_{CO2}}}{\dot{RQ}}$$

$$(11)$$

$$P_{A_{O2}} = P_{I_{O2}} + (1 - R_G)\frac{P_{a_{CO2}}F_{I_{O2}}}{R_G} - \frac{P_{a_{CO2}}}{\dot{RQ}}$$

$$(12)$$

In order to more correctly determine $O_2$-$CO_2$ partial pressure in alveolar gas, there is required a numerical analysis comprising an algorithm to estimate the partial pressure with considering gas exchange between alveolar gas and blood, shunt and dead space according to a threecompartment lung model. In order to find $O_2$-$CO_2$ partial pressure in pulmonary alveolar gas simultaneously satisfying different equations such as mass balance equations, shunt equations, dead space relations, respiratory quotient $RQ$ and respiratory exchange ratio R and the like, the same calculation must be repeated so many times and a complicated calculation is required to estimate blood concentration from $O_2$-$CO_2$ partial pressure. The numerical analysis is very useful for both of the above calculations.

For estimation of $O_2$-$CO_2$ partial pressure in alveolar gas through the numerical analysis comprising the algorithm described above, different input values are needed, which include, for example, mixed venous blood, arterial blood, $O_2$-$CO_2$ partial pressure of inspiration air and/or expiration air, etc. Especially, the mixed venous blood is often sampled by inserting a catheter from superior or inferior vena cava to pulmonary artery through the right heart. Such a catheter operation generally causes severe pain and danger to patients, can be achieved only by a clinical pathologist with high technical skills in the art and, occasionally, may cause complications of patients when the catheter is inserted for a long time. Therefore, this is a difficult medical treatment and restrictedly used for patients with severe respiratory diseases hospitalized in intensive care units or an emergency case. If $O_2$-$CO_2$ partial pressure of the alveolar gas can be predicted even without sampling of the mixed venous blood, the above problems can be overcome and medical charges may be favorably reduced.

$O_2$-$CO_2$ partial pressures in both the mixed venous blood and the alveolar gas may be determined by firstly selecting an area possibly having the mixed venous blood, substituting a specific $O_2$-$CO_2$ partial pressure in the selected area for an algorithm, obtaining $O_2$-$CO_2$ partial pressures in the alveolar gas that satisfy any mathematical equation such as mass balance equation, shunt equation, dead space relation, respiratory quotient $RQ$ and respiratory exchange ratio $R_B$ and $R_G$, etc., selecting desired $O_2$-$CO_2$ partial pressures having minimum difference between $R_B$ and $R_G$, and determining the selected values as the $O_2$-$CO_2$ partial pressures in both the mixed venous blood and the alveolar gas. Shunt rate equations are represented by the following equations Eq. 13 and Eq. 14, dead space rate equations are represented by the following equations Eq. 15 and Eq. 16, ventilation-perfusion ratio equations are represented by the following equations Eq. 17 to Eq. 20 and, finally, cardiac output equations are represented by the following equations Eq. 21 and 22.

Shunt rate equations:

$$Y_{O_2} = (\dot{Q}_S / \dot{Q}_T)_{O_2} = (C_{C_{O_2}} - C_{a_{O_2}}) / (C_{C_{O_2}} - C_{\bar{V}_{O_2}}) \tag{13}$$

$$Y_{CO_2} = (\dot{Q}_S / \dot{Q}_T)_{CO_2} = (C_{a_{CO_2}} - C_{C_{CO_2}}) / (C_{\bar{V}_{CO_2}} - C_{C_{CO_2}}) \tag{14}$$

Dead space rate equations:

$$X_1 = \dot{V}_D / \dot{V}_T = (P_{A_{O_2}} - P_{ET_{O_2}}) / (P_{A_{O_2}} - P_{I_{O_2}}) \tag{15}$$

$$X_2 = \dot{V}_D / \dot{V}_T = (P_{A_{CO_2}} - P_{ET_{CO_2}}) / (P_{A_{CO_2}} - P_{I_{CO_2}}) \tag{16}$$

Ventilation-perfusion ratio equations:

$$(\dot{V}_A / \dot{Q})_1 = \dot{V}_A \times (C_{C_{O_2}} - C_{\bar{V}_{O_2}}) / \dot{V}_{O_2} \tag{17}$$

$$(\dot{V}_A / \dot{Q})_2 = \dot{V}_A \times (C_{\bar{V}_{CO_2}} - C_{C_{CO_2}}) / \dot{V}_{CO_2} \tag{18}$$

$$(\dot{V}_A / \dot{Q})_3 = ((\dot{V}_I / \dot{Q}) P_{I_{O_2}} - \kappa \times (C_{C_{O_2}} - C_{\bar{V}_{O_2}})) / P_{A_{O_2}} \tag{19}$$

$$\left( \dot{V}_A / \dot{Q} \right)_4 = \kappa \times \left( C_{\overline{V}_{CO_2}} - C_{C_{O_2}} \right) / P_{A_{CO_2}}$$

$$(20)$$

Cardiac output equations:

$$\dot{Q}_T = \frac{\dot{Q}}{(1 - Y)}$$

$$(21)$$

$$\dot{Q}_T = \frac{(1 - X)\dot{V}_I}{(1 - Y)\left[\dot{V}_A / \dot{Q}\right]}$$

$$(22)$$

Hereinafter, a method for non-invasively determining pulmonary characteristics by measuring respiratory gas and blood gas, as well as an apparatus for displaying pulmonary characteristics will be described in detail with the accompanying drawings.

Fig. 8 is a block diagram illustrating an apparatus for determining respiratory characteristics in a lungs-pulmonary circulation system according to an exemplary embodiment of the present invention. The apparatus comprises a mask and a nozzle 1 and 2 for passing ventilation air; sensor measuring means 3, 4 and 5 for measuring flow rate and shot partial pressure of the ventilation air from bio-sensors mounted on the nozzle; a micro-processor or computer 7 with a built-in program for analysis of three kinds of respiration model to estimate physiological characteristics of lung-pulmonary circulation system by using primary measurement parameters such as the flow rate and the shot partial pressure; and a means 8 for visibly displaying the primary measurement parameters and estimated physiological characteristics on liquid crystal displays, computer terminals, printers, mobile phones, PDAs, etc. The method for non-invasively determining pulmonary characteristics by measuring respiratory gas and blood gas according to the present invention may be achieved by the above apparatus illustrated in Fig. 8. Hereinafter, the inventive method for non-invasively determining pulmonary characteristics through measurement of respiratory gas and blood gas will be more apparent from the following description.

The apparatus for determining respiratory characteristics in lungs-pulmonary circulation system described above can simultaneously determine physiological characteristics in cardiopulmonary organs including, for example: blood respiratory characteristics such as shot partial pressure of mixed venous blood and/or peripheral capillary blood (or alveolar gas); cardiac functional characteristics such as cardiac output; lung functional characteristics such as shunt rate or dead space rate, and the like.

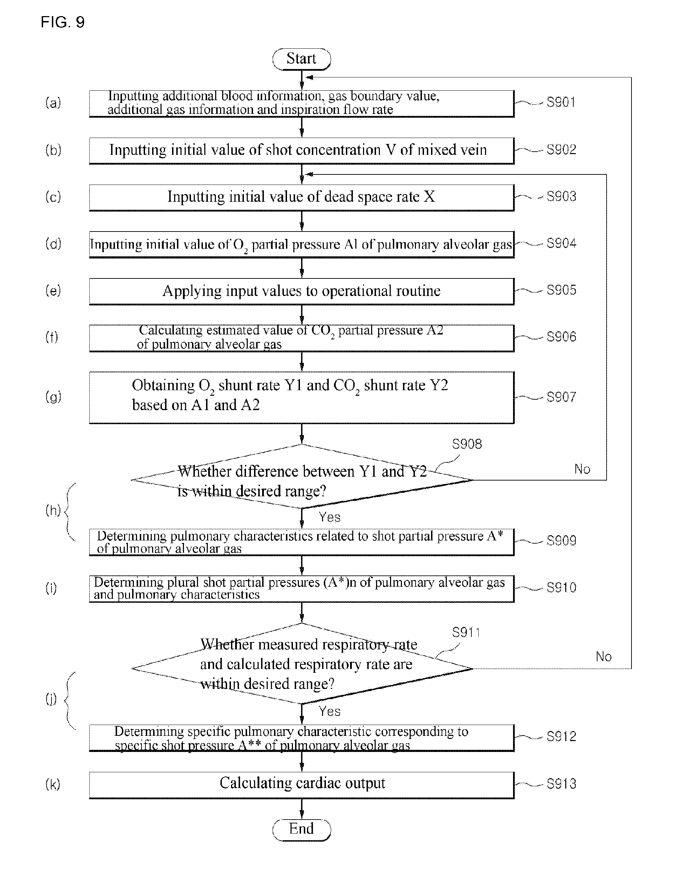

The method for determining pulmonary characteristics according to the present invention may comprise: passing ventilation gas through a nozzle; measuring flow rate of inspired air $V_I$ or expired air, shot partial pressure I* thereof, and/or shot partial pressure of gas at the end of expiration ET* and using these values as primary parameters of respiratory gas; measuring shot partial concentration of arterial blood a*; and inputting the measured parameters into mathematical equations such as mass balance equations related to $O_2$ and $CO_2$ and solving the equations to obtain important physiological characteristics of cardiopulmonary organs. The step of obtaining physiological characteristics will be described in greater detail by the following description with reference to Figs. 9 to 12.

The physiological characteristics includes respiratory functional characteristics such as shot partial pressures of mixed venous blood and/or peripheral capillary blood (or commonly known as pulmonary alveolar gas); cardiac functional characteristics such as cardiac output; and structural characteristics of lungs such as shunt rate and physiological dead space rate, which all are output parameters.

In addition, partial pressures and concentrations of $O_2$ and $CO_2$ which are combined with and/or separated from hemoglobin in red blood cells and diffused into blood and respiratory air reach an inter-equilibrium in pulmonary alveolus and peripheral capillary, and are thereby capable of being changed into each other through gas dissociation curve.

Primary measurement parameters entered as input data have less numerical values but require many pulmonary characteristics to be determined. Accordingly, the present invention may construct an analysis system using different mathematical equations including, for example, mass balance equations for $O_2$ and/or $CO_2$, shunt rate equations for $O_2$ and/or $CO_2$, respiratory rate equation of ventilation air, respiratory rate equation of blood, ventilation-perfusion ratio equation for $O_2$ and/or $CO_2$ and the like.

In order to obtain many pulmonary characteristics using less primary measurement parameters, an aspect of the present invention is to adopt a systematic method to solve complicated problems, comprising: sequentially aligning some problems from ones having a large number of input parameters and a small number of output parameters to others having a small number of input parameters and a large number of output parameters; classifying the problems into three kinds of respiration model; and applying analysis solutions and results induced from a simple one of the problem models to the more complicated problem model.

'First respiration model' relates to a lung without shunt or physiological dead space, which comprises shot partial pressure of mixed vein V* and shot partial pressure of inspired gas I*. 'Second respiration model' relates to a lung with shunt or physiological dead space, which comprises information of mixed venous blood V*, shot partial pressure of arterial blood a*, shot partial pressure of inspired gas I* and shot partial pressure of gas at the end of expiration ET*. 'Third respiration model' relates to a lung with shunt or physiological dead space, which comprises gas boundary value I*, shot partial pressure of gas at the end of expiration ET* as additional gas information and shot partial pressure of arterial blood a*, however, includes shot partial concentration of mixed vein V* as an unknown variable. This respiration model is the most practical one in terms of respiratory physiology for a human body, but there is difficulty in solving the same.

The first respiration model is solved by means of ventilation-perfusion ratio curve or Kelman's curve, and a method for analysis of the first respiration model is briefly described as follows.

The analysis method comprises: at first, forming an outer do-loop and inputting an initial value $\dot{V}_A/\dot{Q}$ of ventilation-perfusion ratio into the loop; forming an inner do-loop and inputting an initial value of $O_2$ partial pressure information of pulmonary alveolar gas a1 into the loop; solving a mass balance equation to calculate $CO_2$ partial pressure a2; and using a pair of the values A* (a1, a2) to obtain another value $(\dot{V}_A/\dot{Q})$* for ventilation-perfusion ratio. When the value $\dot{V}_A/\dot{Q}$ is equal to the value $(\dot{V}_A/\dot{Q})$* as a requirement for ventilation-perfusion ratio, A* is taken as a solution to escape the inner do-loop. While renewing the initial value of $\dot{V}_A/\dot{Q}$ in the outer do-loop, the above calculation process is repeated to offer a Kelman's curve comprising a set of shot partial pressures of pulmonary alveolus matching regularly increased $\dot{V}_A/\dot{Q}$ values, which is often called a ventilation-perfusion ratio curve or $O_2$-$CO_2$ diagram.

However, each of 'second respiration model' and 'third respiration model' having shunt and physiological dead space in a lung includes more unknown variables and/or output parameters than the 'first respiration model', thus necessarily demanding extended analysis procedures. The 'second respiration model' comprises information of mixed vein V*, information of arterial blood a*, information of inspired gas I* and information of gas at the end of expiration ET*. As described above, a method for analysis of the second respiration model comprises: at first, forming an outer do-loop and setting up an initial value of dead space rate X; inputting an initial value of $O_2$ partial pressure of pulmonary alveolar gas a1 into an inner do-loop and solving a mass balance equation system to calculate $CO_2$ partial pressure of pulmonary alveolar gas a2; using A*(a1, a2) to calculate $O_2$ shunt rate Y1 by an equation Eq. 6 and calculate $CO_2$ shunt rate Y2 by an equation Eq. 7, and determining whether these values are the same as each other as a requirement for shunt rate; if the result does not satisfy the requirement for shunt rate, returning to the start of outer do-loop to renew the initial value for dead space rate X and repeating the above calculation; if the result satisfies the requirement for shunt rate, releasing out of both the inner do-loop and the outer do-loop and taking shot partial pressure of pulmonary alveolar gas A*, shunt rate Y* and physiological dead space rate X* from the finally stored values in a memory device as final solutions; and applying inputted inspiration capacity ($V_I$), calculated dead space rate X* and shunt rate Y* to determine cardiac output $Q_{total}$ according to a cardiac output equation Eq. 14 or Eq. 15.

Lastly, the 'third respiration model' with shunt and physiological dead space comprises information of inspired gas I* and information of gas at the end of expiration ET* as well as shot gas partial pressure of arterial blood a*, however, does not have information of mixed vein V*. Accordingly, compared to the second respiration model, the third respiration model has reduced input parameters and increased output parameters. The method for determining pulmonary characteristics according to the present invention may be classified into four types according to initial values inputted into an automatic input device, which are described in detail as follows.

Fig. 9 is a flow chart illustrating a method for determining pulmonary characteristics according to a first type of a third respiration model in the exemplary embodiment of the present invention. Referring to Fig. 9, the method for determining pulmonary characteristics according to the first type model comprises:

(a) inputting respiratory input parameters such as additional blood information, gas boundary value, additional gas information, inspiration flow rate, etc. into an automatic operation device (S901). The automatic operation device may comprise a microprocessor or a computer device 7 with a built-in program for analysis of three kinds of respiration model as illustrated in Fig. 8;

The additional blood information may comprise shot partial pressure of arterial blood a* and/or measured $O_2$ partial pressure thereof, while the gas boundary value may comprise shot partial pressure of inspired gas I* or measured $O_2$ partial pressure thereof.

The additional gas information may comprise shot partial pressure of gas at the end of expiration ET*, especially, $CO_2$ partial pressure, while the inspiration flow rate $\dot{V_I}$ is the total flow rate of external air supplied to lungs, which may be defined to be substantially the same as expiration flow rate VE which is the total flow rate released from the lungs.

Next, (b) inputting an initial value of shot concentration of mixed vein V into the automatic operation device (S902); and (c) inputting an initial value of dead space rate X into the automatic operation device (S903). In the present invention, the order of inputting the respiratory input parameters and the above initial values is not particularly limited.

Following this, (d) inputting an initial value of $O_2$ partial pressure of pulmonary alveolar gas A1 based on the initial value of dead space rate X as respiratory input parameters into the automatic operation device (S904); and

(e) applying the respiratory input parameters and the inputted initial values to an operational routine provided in the automatic operation device, so as to obtain a solution from equations for respiratory gas including mass balance equations for 02, CO2 and/or N2 as well as Fick's equation (S905).

Thereafter, (f) calculating an estimated value of $CO_2$ partial pressure of pulmonary alveolar gas A2 as a solution obtained from analytical equations for respiratory gas via the operational routine (S906); and (g) calculating $O_2$ shunt rate Y1 and $CO_2$ shunt rate Y2 based on the initial value of $O_2$ partial pressure and the estimated $CO_2$ partial pressure of pulmonary alveolar gas A1 and A2 (S907). The analytical equation for respiratory gas may comprise mass balance equations for $O_2$, $CO_2$ and/or $N_2$.

Next, (h) if shunt rate requirements are satisfied (Yes in S908), determining pulmonary characteristics related to shot partial pressure of pulmonary alveolar gas A* (S909). The shunt rate requirements are to compare and determine whether a difference between the $O_2$ shunt rate Y1 and the $CO_2$ shunt rate Y2 is within a desired range. If the requirements are not satisfied (No in S908), the inventive method includes returning to the step (c) and repeatedly carrying out the above steps.

Subsequently, (i) repeating steps (b) to (h) with plural shot partial pressures of mixed vein (V*)n at a constant interval as initial values so as to determine plural shot partial pressures of pulmonary alveolar gas (A*)n corresponding to the plural shot partial pressures of mixed vein (V*)n, respectively, as well as pulmonary characteristics related thereto (S910).

The plural shot partial pressures of mixed vein (V*)n correspond to lattice points of a plurality of divided "mixed vein lattices", respectively, each being setup as a blood boundary value and inputted into the automatic operation device. The lattice points of the plural divided "mixed vein lattices" may have irregular spaces between lattices and comprise multi-grids including larger and smaller grids combined together, especially, a primary larger grid capable of being further re-divided into smaller ones.

Continuously, (j) when a specific shot pressure of pulmonary alveolar gas A** satisfying requirements for respiratory rate is selected from the plural shot partial pressures of pulmonary alveolar gas (Yes in S911), determining a specific pulmonary characteristic corresponding to the specific shot pressure of pulmonary alveolar gas A** (S912); and (k) calculating cardiac output (S913).

The requirements for respiratory rate are to compare and determine whether a difference between a respiratory rate obtained from calculated numerical values and another respiratory rate obtained from the difference between shot partial pressures of inspired gas and gas at the end of expiration is within a desired range. If the specific shot pressure of pulmonary alveolar gas A** to satisfy the requirements for respiratory rate is not defined, the inventive method includes returning to the step (a) and repeatedly carrying out the above steps.

The specific pulmonary characteristic determined in step (j) means any one selected from shot partial pressure of pulmonary alveolar gas A**, shot partial pressure of peripheral capillary blood C**, ventilation-perfusion ratio ($\dot{V_A}/\dot{Q}$) **, shunt rate Y** and physiological dead space rate X**. The cardiac output in step (k) may be calculated using measured amount of inspired air $\dot{V_I}$ or of expired air VE and the physiological dead space rate X**.

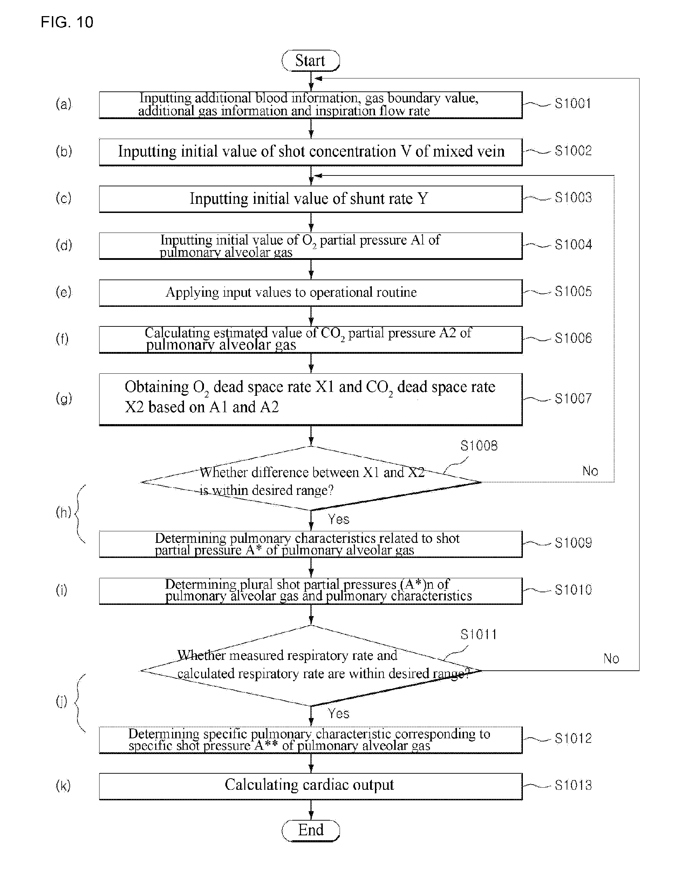

Fig. 10 is a flow chart illustrating a method for determining pulmonary characteristics according to a second type of the third respiration model in the exemplary embodiment of the present invention. Referring to Fig. 10, the method for determining pulmonary characteristics according to the second type of the third respiration model preferably comprises:

(a) inputting respiratory input parameters including additional blood information, gas boundary value, additional gas information, inspiration flow rate, etc. into an automatic operation device (S1001); (b) inputting an initial value of shot concentration of mixed vein V into the automatic operation device (S1002); (c) inputting an initial value of shunt rate Y into the automatic operation device (S1003); (d) inputting an initial value of $O_2$ partial pressure of pulmonary alveolar gas A1 based on the initial value of shunt Y as respiratory input parameters into the automatic operation device (S1004); (e) applying the respiratory input parameters and the inputted initial values to an operational routine provided in the automatic operation device, so as to obtain a solution from equations for respiratory gas including mass balance equations for 02, CO2 and/or N2 as well as Fick's equation (S1005); (f) calculating an estimated value of $CO_2$ partial pressure of pulmonary alveolar gas A2 as a solution obtained from analytical equations for respiratory gas via the operational routine (S1006); (g) calculating $O_2$ dead space rate X1 and $CO_2$ dead space rate X2 based on the initial value of $O_2$ partial pressure and the estimated $CO_2$ partial pressure of pulmonary alveolar gas A1 and A2 (S1007); (h) if dead space rate requirements are satisfied (Yes in S1008), determining pulmonary characteristics related to shot partial pressure of pulmonary alveolar gas A* (S1009); (i) repeating steps (b) to (h) with plural shot partial pressures of mixed vein (V*)n at a constant interval as initial values so as to determine plural shot partial pressures of pulmonary alveolar gas (A*)n corresponding to the plural shot partial pressures of mixed vein (V*)n, respectively, as well as pulmonary characteristics related thereto (S1010); (j) when a specific shot pressure of pulmonary alveolar gas A** satisfying requirements for respiratory rate is selected from the plural shot partial pressures of pulmonary alveolar gas (A*)n (S1011), determining a specific pulmonary characteristic corresponding to the specific shot pressure of pulmonary alveolar gas A** (S1012); and (k) calculating cardiac output (S1013).

The method for determining pulmonary characteristics according to the above second type of the third respiration model is substantially similar to the method according to the first type of the third respiration model shown in Fig. 9, therefore, a detailed description is omitted to avoid the subject matter of the invention being duplicated. A difference between both the models is that step (c) of the first type model comprises inputting initial value of dead space rate X whereas step (c) in the second type model is to input initial value of shunt rate Y. In addition, in step (h) of the second type model, the dead space rate requirements are to compare and determine whether a difference between the $O_2$ dead space rate X1 and the $CO_2$ dead space rate X2 is within a desired range. If the dead space rate requirements are not satisfied (No in S1008), the inventive method includes returning to the step (c) and repeatedly carrying out the above steps.

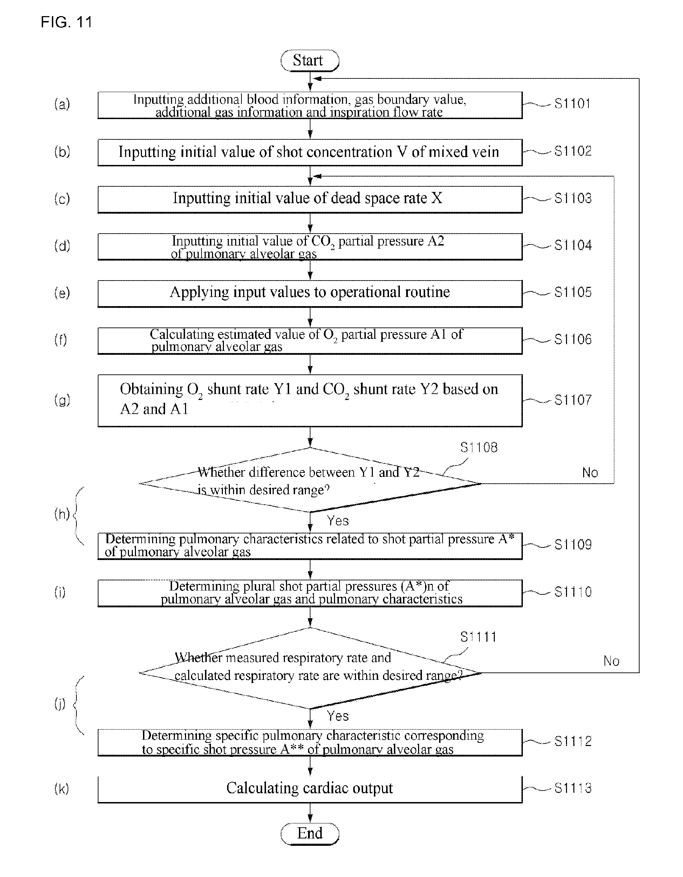

Fig. 11 is a flow chart illustrating a method for determining pulmonary characteristics according to a third type of the third respiration model in the exemplary embodiment of the present invention. Referring to Fig. 11, the method for determining pulmonary characteristics according to the third type of the third respiration model preferably comprises:

(a) inputting respiratory input parameters including additional blood information, gas boundary value, additional gas information, inspiration flow rate, etc. into an automatic operation device (S1101); (b) inputting an initial value of shot concentration of mixed vein V into the automatic operation device (S1102); (c) inputting an initial value of dead space rate X into the automatic operation device (S1103); (d) inputting an initial value of $CO_2$ partial pressure of pulmonary alveolar gas A2 based on the initial value of dead space rate X as respiratory input parameters into the automatic operation device (S1104); (e) applying the respiratory input parameters and the inputted initial values to an operational routine provided in the automatic operation device, so as to obtain a solution from equations for respiratory gas including mass balance equations for 02, CO2 and/or N2 as well as Fick's equation (S1105); (f) calculating an estimated value of $O_2$ partial pressure of pulmonary alveolar gas A1 as a solution obtained from analytical equations for respiratory gas via the operational routine (S1106); (g) calculating $O_2$ shunt rate Y1 and $CO_2$ shunt rate Y2 based on the initial value of $CO_2$ partial pressure and the estimated $O_2$ partial pressure of pulmonary alveolar gas A2 and A1 (S1107); (h) if shunt rate requirements are satisfied (Yes in S1108), determining pulmonary characteristics related to shot partial pressure of pulmonary alveolar gas A* (S1109); (i) repeating steps (b) to (h) with plural shot partial pressures of mixed vein (V*)n at a constant interval as initial values so as to determine plural shot partial pressures of pulmonary alveolar gas (A*)n corresponding to the plural shot partial pressures of mixed vein (V*)n, respectively, as well as pulmonary characteristics related thereto (S1110); (j) when a specific shot pressure of pulmonary alveolar gas A** satisfying requirements for respiratory rate is selected from the plural shot partial pressures of pulmonary alveolar gas (A*)n (S1111), determining a specific pulmonary characteristic corresponding to the specific shot pressure of pulmonary alveolar gas A** (S1112); and (k) calculating cardiac output (S1113).

The method for determining pulmonary characteristics according to the above third type of the third respiration model is substantially similar to the method according to the first type of the third respiration model shown in Fig. 9, therefore, a detailed description is omitted to avoid the subject matter of the invention being duplicated. Differences between both the models are that step (d) of the first type model comprises inputting initial value of $CO_2$ partial pressure of pulmonary alveolar gas A2 on the initial value of dead space rate X as respiratory input parameters into the automatic operation device and step (f) thereof comprises calculating estimated value of $CO_2$ partial pressure of alveolar gas A2 as a solution of analytical equations for respiratory gas via an operational routine, whereas step (d) of the third type model comprises inputting initial value of $CO_2$ partial pressure of pulmonary alveolar gas A2 based on the initial value of dead space rate X as respiratory input parameters into the automatic operation device and step (f) thereof comprises calculating estimated value of $O_2$ partial pressure of alveolar gas A1 as a solution of analytical equations for respiratory gas via an operational routine.

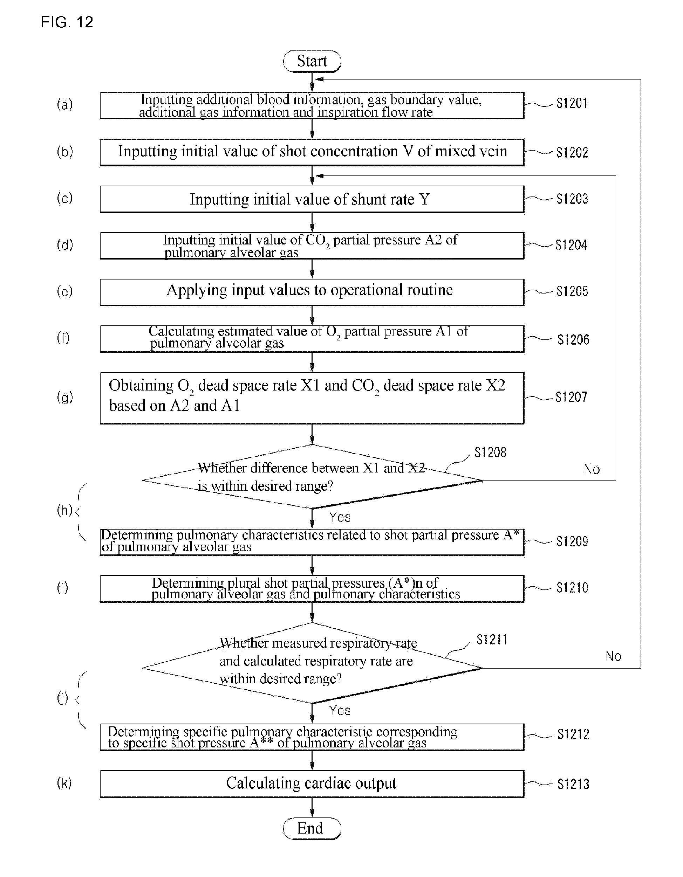

Fig. 12 is a flow chart illustrating a method for determining pulmonary characteristics according to a fourth type of the third respiration model in the exemplary embodiment of the present invention. Referring to Fig. 12, the method for determining pulmonary characteristics according to the fourth type of the third respiration model preferably comprises:

(a) inputting respiratory input parameters including additional blood information, gas boundary value, additional gas information, inspiration flow rate, etc. into an automatic operation device (S1201); (b) inputting an initial value of shot concentration of mixed vein V into the automatic operation device (S1202); (c) inputting an initial value of shunt rate Y into the automatic operation device (S1203); (d) inputting an initial value of $CO_2$ partial pressure of pulmonary alveolar gas A2 based on the initial value of dead space rate Y as respiratory input parameters into the automatic operation device in consideration of the inputted initial value of shunt rate Y (S1204); (e) applying the respiratory input parameters and the inputted initial values to an operational routine provided in the automatic operation device, so as to obtain a solution from equations for respiratory gas including mass balance equations for $O_2$, $CO_2$ and/or $N_2$ as well as Fick's equation (S1205); (f) calculating an estimated value of $O_2$ partial pressure of pulmonary alveolar gas A1 as a solution obtained from analytical equations for respiratory gas via the operational routine (S1206); (g) calculating $O_2$ dead space rate X1 and $CO_2$ dead space rate X2 based on the initial value of $CO_2$ partial pressure and the estimated $O_2$ partial pressure of pulmonary alveolar gas A2 and A1 (S1207); (h) if dead space rate requirements are satisfied (Yes in S1208), determining pulmonary characteristics related to shot partial pressure of pulmonary alveolar gas A* (S1209); (i) repeating steps

(b) to (h) with plural shot partial pressures of mixed vein (V*)n at a constant interval as initial values so as to determine plural shot partial pressures of pulmonary alveolar gas (A*)n corresponding to the plural shot partial pressures of mixed vein (V*)n, respectively, as well as pulmonary characteristics related thereto (S1210); (j) when a specific shot pressure of pulmonary alveolar gas A** satisfying requirements for respiratory rate is selected from the plural shot partial pressures of pulmonary alveolar gas (A*)n (Yes in S1211), determining a specific pulmonary characteristic corresponding to the specific shot pressure of pulmonary alveolar gas A** (S1212); and (k) calculating cardiac output (S1213).

The method for determining pulmonary characteristics according to the above fourth type of the third respiration model is substantially similar to the method according to the second type of the third respiration model shown in Fig. 10, therefore, a detailed description is omitted to avoid the subject matter of the invention being duplicated.

Differences between both the models are that step (d) of the second type model comprises inputting initial value of $O_2$ partial pressure of alveolar gas A1 based on the initial value of shunt rate Y as respiratory input parameters into the automatic operation device and step (f) thereof comprises calculating estimated value of $CO_2$ partial pressure of alveolar gas A2 as a solution of analytical equations for respiratory gas via an operational routine, whereas step (d) of the fourth type model comprises inputting initial value of $CO_2$ partial pressure of alveolar gas A2 based on the initial value of shunt rate Y as respiratory input parameters into the automatic operation device and step (f) thereof comprises calculating estimated value of $O_2$ partial pressure of alveolar gas A1 as a solution of analytical equations for respiratory gas via an operational routine.

In step (a) of each of the first, second, third and fourth type models, the additional blood information only comprises shot $O_2$ partial pressure of arterial blood a1*, while shot $CO_2$ partial pressure of arterial blood a2* is defined by repeating steps (a) to (k).

An apparatus for displaying pulmonary characteristics according to the present invention includes an information terminal connected to an automatic operation device to visibly display pulmonary characteristics determined by any one selected from first, second, third and fourth type methods for determining pulmonary characteristics according to the present invention.

The apparatus for displaying pulmonary characteristics described above has a portable type information terminal capable being wired/wirelessly connected to the automatic operation device, wherein the automatic operation device may comprise computer processors and/or embedded chips provided in a computer.

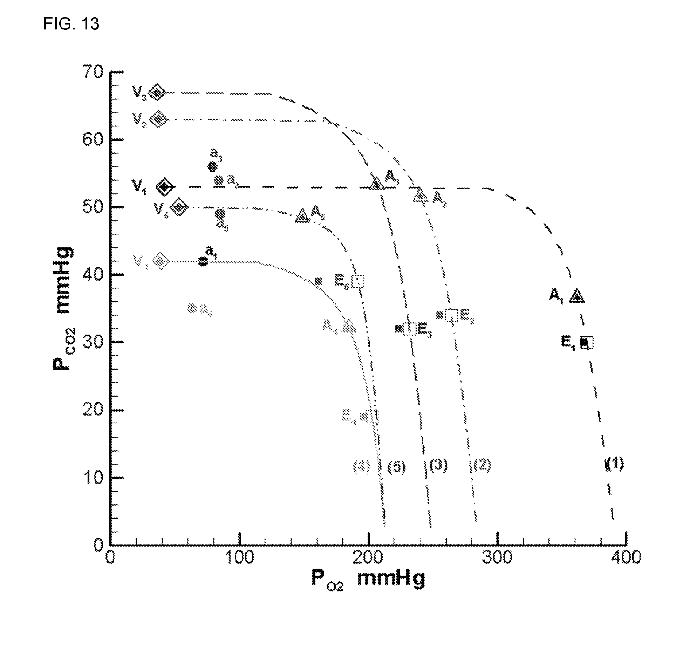

Fig. 13 is graphs illustrating ventilation-perfusion ratio curves according to an exemplary embodiment of the present invention. Curves shown in Fig. 7 (1, 2, 3, 4, and 5) are ventilation-perfusion ratio curves resulted from solutions of 'first respiration model' problems for five patients. A symbol with a large "diamond shape" at the left end of each of the curves (V1, V2, ..., and V5) represents measured shot partial pressure value of mixed vein V* among the clinical data set M. Also, a small and black "diamond shape" inside the large diamond shape is concentration data of mixed vein V** obtained by calculating 'third respiration model' problems and is clearly demonstrated to correctly match V* point.

A black circle (a1, a2, ..., and a5) near the diamond symbol represents shot partial pressure of arterial blood a* among clinical data set M. A large triangle at center portion of each of the curves (A1 and A2, ..., and A5) is partial pressure of pulmonary alveolar gas A* which was defined by using V*, a*, I*, and ET* among the clinical data set M as well as analytical solutions of 'second respiration model' according to the present invention. Also, a small and black triangle inside the large triangle is partial pressure of alveolar gas A** calculated by analytical solutions of 'third respiration model' according to the present invention and is clearly demonstrated to correctly match A*.

A blank square (E1, E2, ..., and E5) represents $CO_2$ partial pressure of gas at the end of expiration contained in the clinical data set M marked on the ventilation-perfusion ratio curve obtained from 'first respiration model', while small and black squares near the blank square correctly indicate shot partial pressure points of gas at the end of expiration ET* by further applying $O_2$ partial pressure of gas at the end of expiration calculated from analytical results of 'second respiration model' according to the present invention. These values are in turn used as shot partial pressure information of gas at the end of expiration ET*, which is required to analyze 'third respiration model' of the present invention.

Consequently, from tables demonstrating the above calculated values compared with known clinical values, it is clearly understood that the systematic respiration analysis method according to the present invention can accurately predict and/or determine physiological characteristics of cardiopulmonary organs.

While the present invention has been described with reference to the limited embodiments and accompanying drawings, these are given to illustrate the purposes and technical constructions of the present invention but do not limit the scope of the present invention. It will be understood by those skilled in the art that various modifications and variations may be made therein without departing from the scope of the present invention.

Accordingly, the spirit of the present invention is not restricted to exemplary embodiments described herein and the scope of the present invention duly includes various changes and modifications equivalent to subject matters as defined by the appended claims.

## Claims

**1.** A method for determining pulmonary characteristics, comprising:

(a) inputting respiratory input parameters including additional blood information, gas boundary value, additional gas information, inspiration flow rate, etc. into an automatic operation device;

(b) inputting an initial value of shot concentration of mixed vein V into the automatic operation device;

(c) inputting an initial value of dead space rate X into the automatic operation device;

(d) inputting an initial value of $O_2$ partial pressure of pulmonary alveolar gas A1 based on the initial value of dead space rate X as respiratory input parameters into the automatic operation device;

(e) applying the respiratory input parameters and the inputted initial values to an operational routine provided in the automatic operation device, so as to obtain a solution from equations for respiratory gas including mass balance equations for O2, CO2 and/or N2 as well as Fick's equation;

(f) calculating an estimated value of $CO_2$ partial pressure of pulmonary alveolar gas A2 as a solution obtained from analytical equations for respiratory gas via the operational routine;

(g) calculating $O_2$ shunt rate Y1 and $CO_2$ shunt rate Y2 based on the initial value of $O_2$ partial pressure and the estimated $CO_2$ partial pressure of pulmonary alveolar gas A1 and A2;

(h) determining pulmonary characteristics related to shot partial pressure of pulmonary alveolar gas A*, when shunt rate requirements are satisfied;

(i) repeating steps (b) to (h) with plural shot partial pressures of mixed vein (V*)n at a constant interval as initial values so as to determine plural shot partial pressures of pulmonary alveolar gas (A*)n corresponding to the plural shot partial pressures of mixed vein (V*)n, respectively, as well as pulmonary characteristics related thereto;

(j) when a specific shot pressure of pulmonary alveolar gas A** satisfying requirements for respiratory rate is selected from the plural shot partial pressures of pulmonary alveolar gas (A*)n, determining a specific pulmonary characteristic corresponding to the specific shot pressure of pulmonary alveolar gas A**; and

(k) calculating cardiac output.

**2.** The method according to claim 1, wherein the additional blood information in step (a) is shot partial pressure of arterial blood a* or measured $O_2$ partial pressure thereof.

**3.** The method according to claim 1,

wherein the gas boundary value in step (a) is shot partial pressure of inspired gas I* or measured $O_2$ partial pressure thereof.

4. The method according to claim 1,
   wherein the additional gas information in step (a) is shot partial pressure of gas at the end of expiration ET* or measured $CO_2$ partial pressure thereof.

5. The method according to claim 1,
   wherein the inspiration flow rate VI in step (a) is total flow rate of external air received by lungs and is setup to be substantially the same level as an expiration flow rate VE that is total flow rate of air released from the lungs.

6. The method according to claim 1,
   wherein the shunt rate requirements in step (h) are to compare and determine whether a difference between the $O_2$ shunt rate Y1 and the $CO_2$ shunt rate Y2 is within a desired range.

7. The method according to claim 1,
   wherein the analytic equations for respiratory gas in step (f) include the following equations (1), (2) and (3) as mass balance equations for $O_2$, $CO_2$ and/or $N_2$, respectively:

$$\left( \dot{V_I}/\dot{Q} \right) P_{I_{O2}} - \left( \dot{V_A}/\dot{Q} \right) P_{A_{O2}} = \kappa \times \left( C_{C_{O2}} - C_{\overline{V}_{O2}} \right) \qquad (1)$$

$$\left( \dot{V_A}/\dot{Q} \right)_2 P_{A_{CO2}} = \kappa \left( C_{\overline{V}_{CO2}} - C_{C_{CO2}} \right) \qquad (2)$$

$$\left( \dot{V_I}/\dot{Q} \right)_3 P_{I_{N2}} - \left( \dot{V_A}/\dot{Q} \right)_3 P_{A_{N2}} = \lambda \left( P_{C_{N2}} - P_{\overline{V}_{N2}} \right) \qquad (3)$$

(wherein,

$C_{CCO2}$ : $CO_2$ concentration in capillary [%]
$C_{CCO2}$ : $O_2$ concentration in mixed vein [%]
$C_{\overline{V}CO2}$ : $CO_2$ concentration in mixed vein [%]
$C_{\overline{V}O2}$ : $O_2$ concentration in mixed vein [%]
$P_{ACO2}$ : $CO_2$ partial pressure in pulmonary alveolus [mmHg]
$P_{AN2}$ : $N_2$ partial pressure in pulmonary alveolus [mmHg]
$P_{AO2}$ : $O_2$ partial pressure in pulmonary alveolus [mmHg]
$P_{CN2}$ : $N_2$ partial pressure of capillary [mmHg]
$P_{IN2}$ : **$N_2$ partial pressure of air in atmosphere [mmHg]**
$P_{IO2}$ : $O_2$ partial pressure of air in atmosphere [mmHg]
$\overline{P}_{VN2}$ : $N_2$ partial pressure of mixed vein [mmHg],
$\dot{Q}$: perfusion of capillary [liters/min]
$\dot{V}_A$ : flow rate of air gas-exchanged in pulmonary alveolus [liters/min]
$\dot{V}_I$ : flow rate of inspiration air [liters/min]
$\kappa$ : respiratory quotient
$\lambda$ : constant for blood and air)

8. The method according to claim 1,
   wherein the plural shot partial pressures of mixed vein (V*)n in step (i) correspond to lattice points of a plurality of divided "mixed vein lattices", respectively, each being setup as a blood boundary value and inputted into the automatic operation device.

9. The method according to claim 8, wherein the lattice points of the plurality of divided "mixed vein lattices" have irregular spaces between lattices, comprise multi-grids including larger and smaller grids combined together and, optionally, a primary larger grid capable of being further re-divided into smaller ones.

10. A method for determining pulmonary characteristics, comprising:

(a) inputting respiratory input parameters including additional blood information, gas boundary value, additional gas information, inspiration flow rate, etc. into an automatic operation device;
(b) inputting an initial value of shot concentration of mixed vein V into the automatic operation device;
(c) inputting an initial value of shunt rate Y into the automatic operation device;
(d) inputting an initial value of $O_2$ partial pressure of pulmonary alveolar gas A1 based on the initial value of shunt Y as respiratory input parameters into the automatic operation device;
(e) applying the respiratory input parameters and the inputted initial values to an operational routine provided in the automatic operation device, so as to obtain a solution from equations for respiratory gas including mass balance equations for 02, CO2 and/or N2 as well as Fick's equation;
(f) calculating an estimated value of $CO_2$ partial pressure of pulmonary alveolar gas A2 as a solution obtained from analytical equations for respiratory gas via the operational routine;
(g) calculating $O_2$ dead space rate X1 and $CO_2$ dead space rate X2 based on the initial value of $O_2$ partial pressure and the estimated $CO_2$ partial pressure of pulmonary alveolar gas A1 and A2;
(h) determining pulmonary characteristics related to shot partial pressure of pulmonary alveolar gas A*, when dead space rate requirements are satisfied,;
(i) repeating steps (b) to (h) with plural shot partial pressures of mixed vein (V*)n at a constant interval as initial values so as to determine plural shot partial pressures of pulmonary alveolar gas (A*)n corresponding to the plural shot partial pressures of mixed vein (V*)n, respectively, as well as pulmonary characteristics related thereto;
(j) when a specific shot pressure of pulmonary alveolar gas A** satisfying requirements for respiratory rate is selected from the plural shot partial pressures of pulmonary alveolar gas (A*)n, determining a specific pulmonary characteristic corresponding to the specific shot pressure of pulmonary alveolar gas A**; and
(k) calculating cardiac output.

11. The method according to claim 10, wherein the dead space rate requirements in step (h) are to compare and determine whether a difference between the $O_2$ dead space rate X1 and the $CO_2$ dead space rate X2 is within a desired range.

12. A method for determining pulmonary characteristics, comprising:

(a) inputting respiratory input parameters including additional blood information, gas boundary value, additional gas information, inspiration flow rate, etc. into an automatic operation device;
(b) inputting an initial value of shot concentration of mixed vein V into the automatic operation device;
(c) inputting an initial value of dead space rate X into the automatic operation device;
(d) inputting an initial value of $CO_2$ partial pressure of pulmonary alveolar gas A2 based on the initial value of dead space rate X as respiratory input parameters into the automatic operation device;
(e) applying the respiratory input parameters and the inputted initial values to an operational routine provided in the automatic operation device, so as to obtain a solution from equations for respiratory gas including mass balance equations for $O_2$, $CO_2$ and/or $N_2$ as well as Fick's equation;
(f) calculating an estimated value of $O_2$ partial pressure of pulmonary alveolar gas A1 as a solution obtained from analytical equations for respiratory gas via the operational routine;
(g) calculating $O_2$ shunt rate Y1 and $CO_2$ shunt rate Y2 based on the initial value of $CO_2$ partial pressure and the estimated $O_2$ partial pressure of pulmonary alveolar gas A2 and A1;
(h) determining pulmonary characteristics related to shot partial pressure of pulmonary alveolar gas A*, when shunt rate requirements are satisfied;
(i) repeating steps (b) to (h) with plural shot partial pressures of mixed vein (V*)n at a constant interval as initial values so as to determine plural shot partial pressures of pulmonary alveolar gas (A*)n corresponding to the plural shot partial pressures of mixed vein (V*)n, respectively, as well as pulmonary characteristics related thereto;

(j) when a specific shot pressure of pulmonary alveolar gas A** satisfying requirements for respiratory rate is selected from the plural shot partial pressures of pulmonary alveolar gas (A*)n, determining a specific pulmonary characteristic corresponding to the specific shot pressure of pulmonary alveolar gas A**; and

(k) calculating cardiac output.

13. The method according to claim 12,
   wherein the shunt rate requirements in step (h) are to compare and determine whether a difference between the $O_2$ shunt rate Y1 and the $CO_2$ shunt rate Y2 is within a desired range.

14. A method for determining pulmonary characteristics, comprising:

   (a) inputting respiratory input parameters including additional blood information, gas boundary value, additional gas information, inspiration flow rate, etc. into an automatic operation device;

   (b) inputting an initial value of shot concentration of mixed vein V into the automatic operation device;

   (c) inputting an initial value of shunt rate Y into the automatic operation device;

   (d) inputting an initial value of $CO_2$ partial pressure of pulmonary alveolar gas A2 based on the initial value of shunt rate Y as respiratory input parameters into the automatic operation device;

   (e) applying the respiratory input parameters and the inputted initial values to an operational routine provided in the automatic operation device, so as to obtain a solution from equations for respiratory gas including mass balance equations for $O_2$, $CO_2$ and/or $N_2$ as well as Fick's equation;

   (f) calculating an estimated value of $O_2$ partial pressure of pulmonary alveolar gas A1 as a solution obtained from analytical equations for respiratory gas via the operational routine;

   (g) calculating $O_2$ dead space rate X1 and $CO_2$ dead space rate X2 based on the initial value of $CO_2$ partial pressure and the estimated $O_2$ partial pressure of pulmonary alveolar gas A2 and A1;

   (h) determining pulmonary characteristics related to shot partial pressure of pulmonary alveolar gas A*, when dead space rate requirements are satisfied;

   (i) repeating steps (b) to (h) with plural shot partial pressures of mixed vein (V*)n at a constant interval as initial values so as to determine plural shot partial pressures of pulmonary alveolar gas (A*)n corresponding to the plural shot partial pressures of mixed vein (V*)n, respectively, as well as pulmonary characteristics related thereto;

   (j) when a specific shot pressure of pulmonary alveolar gas A** satisfying requirements for respiratory rate is selected from the plural shot partial pressures of pulmonary alveolar gas (A*)n, determining a specific pulmonary characteristic corresponding to the specific shot pressure of pulmonary alveolar gas A**; and

   (k) calculating cardiac output.

15. The method according to claim 14,
   wherein the dead space rate requirements in step (h) are to compare and determine whether a difference between the $O_2$ dead space rate X1 and the $CO_2$ dead space rate X2 is within a desired range.

16. The method according to any one of claims 1, 10, 12 and 14,
   wherein the requirements for respiratory rate in step (j) are to compare and determine whether a difference between a respiratory rate obtained from calculated numerical values and another respiratory rate obtained from the difference between shot partial pressures of inspired gas and gas at the end of expiration is within a desired range.

17. The method according to claim 16,
   wherein the specific pulmonary characteristic determined in step (j) is any one selected from shot partial pressure of pulmonary alveolar gas A**, shot partial pressure of peripheral capillary blood C**, ventilation-perfusion ratio ($\dot{V}_A/\dot{Q}$)**, shunt rate Y** and physiological dead space rate X**.

18. The method according to claim 16,
   wherein the cardiac output in step (k) is calculated using measured amount of inspired air $\dot{V}_I$ or of expired air VE and the physiological dead space rate X**.

19. The method according to any one of claims 1, 10, 12 and 14,
   wherein the additional blood information in step (a) only includes shot $O_2$ partial pressure of arterial blood a1*, while shot $CO_2$ partial pressure of arterial blood a2* is determined by repeating steps (a) to (k).

**20.** An apparatus for displaying pulmonary characteristics, comprising an information terminal connected to an automatic operation device to visibly display pulmonary characteristics, which are determined by a method for determining the pulmonary characteristics as set forth in any one of claims 1, 10, 12 and 14.

**21.** The apparatus according to claim 20,
wherein the information terminal is wired or wirelessly connected to the automatic operation device and portably carried.

**22.** The apparatus according to claim 21,
wherein the automatic operation device comprises a computer processor or embedded chips.

FIG. 1

FIG. 2

FIG. 3

FIG. 4

FIG. 5

A: Cardiac output measuring unit

B: ECG measuring unit

FIG. 6

Inspired gas
$P_1O_2$   $P_1CO_2$
$\dot{V}_1 = \dot{V}_A + \dot{V}_D$

$\dot{V}_D$

Dead space
$P_1O_2$
$P_1CO_2$

$\dot{V}_A$

Alveolar gas
$P_AO_2$   $P_ACO_2$

Pulmonary
end-capillary
Blood

$\dot{Q}_T - \dot{Q}_S$

$Pc^1O_2$
$Pc^1CO_2$

$\dot{Q}_S$

$P_VO_2,$   $P_VCO_2$

$P_VO_2,$   $P_VCO_2$
Mixed Venous
Blood

$P_aO_2,$   $P_aCO_2$
Arterial blood

FIG. 7

FIG. 8

FIG. 9

```
                          (  Start  )
                               │
                               ▼
(a)   ┌─────────────────────────────────────────────────┐
      │ Inputting additional blood information, gas boundary value, │──── S901
      │ additional gas information and inspiration flow rate │
      └─────────────────────────────────────────────────┘
                               │
                               ▼
(b)   ┌─────────────────────────────────────────────────┐
      │ Inputting initial value of shot concentration V of mixed vein │──── S902
      └─────────────────────────────────────────────────┘
                               │
                               ▼
(c)   ┌─────────────────────────────────────────────────┐
      │ Inputting initial value of dead space rate X │──── S903
      └─────────────────────────────────────────────────┘
                               │
                               ▼
(d)   ┌─────────────────────────────────────────────────┐
      │ Inputting initial value of O₂ partial pressure Al of pulmonary alveolar gas │──── S904
      └─────────────────────────────────────────────────┘
                               │
                               ▼
(e)   ┌─────────────────────────────────────────────────┐
      │ Applying input values to operational routine │──── S905
      └─────────────────────────────────────────────────┘
                               │
                               ▼
(f)   ┌─────────────────────────────────────────────────┐
      │ Calculating estimated value of CO₂ partial pressure A2 │──── S906
      │ of pulmonary alveolar gas │
      └─────────────────────────────────────────────────┘
                               │
                               ▼
(g)   ┌─────────────────────────────────────────────────┐
      │ Obtaining O₂ shunt rate Y1 and CO₂ shunt rate Y2 │──── S907
      │ based on A1 and A2 │
      └─────────────────────────────────────────────────┘
```

(a) Inputting additional blood information, gas boundary value, additional gas information and inspiration flow rate — S901

(b) Inputting initial value of shot concentration V of mixed vein — S902

(c) Inputting initial value of dead space rate X — S903

(d) Inputting initial value of $O_2$ partial pressure Al of pulmonary alveolar gas — S904

(e) Applying input values to operational routine — S905

(f) Calculating estimated value of $CO_2$ partial pressure A2 of pulmonary alveolar gas — S906

(g) Obtaining $O_2$ shunt rate Y1 and $CO_2$ shunt rate Y2 based on A1 and A2 — S907

(h) S908 — Whether difference between Y1 and Y2 is within desired range? — No

Yes

S909 — Determining pulmonary characteristics related to shot partial pressure A* of pulmonary alveolar gas

(i) S910 — Determining plural shot partial pressures (A*)n of pulmonary alveolar gas and pulmonary characteristics

(j) S911 — Whether measured respiratory rate and calculated respiratory rate are within desired range? — No

Yes

S912 — Determining specific pulmonary characteristic corresponding to specific shot pressure A** of pulmonary alveolar gas

(k) Calculating cardiac output — S913

( End )

FIG. 10

$$\boxed{\text{Start}}$$

(a) | Inputting additional blood information, gas boundary value, additional gas information and inspiration flow rate — S1001

(b) | Inputting initial value of shot concentration V of mixed vein — S1002

(c) | Inputting initial value of shunt rate Y — S1003

(d) | Inputting initial value of $O_2$ partial pressure A1 of pulmonary alveolar gas — S1004

(e) | Applying input values to operational routine — S1005

(f) | Calculating estimated value of $CO_2$ partial pressure A2 of pulmonary alveolar gas — S1006

(g) | Obtaining $O_2$ dead space rate X1 and $CO_2$ dead space rate X2 based on A1 and A2 — S1007

S1008

(h) ⟨ Whether difference between X1 and X2 is within desired range? — No

Yes

Determining pulmonary characteristics related to shot partial pressure A* of pulmonary alveolar gas — S1009

(i) | Determining plural shot partial pressures (A*)n of pulmonary alveolar gas and pulmonary characteristics — S1010

S1011

(j) ⟨ Whether measured respiratory rate and calculated respiratory rate are within desired range? — No

Yes

Determining specific pulmonary characteristic corresponding to specific shot pressure A** of pulmonary alveolar gas — S1012

(k) | Calculating cardiac output — S1013

$$\boxed{\text{End}}$$

FIG. 11

$$\boxed{\text{Start}}$$

(a) | Inputting additional blood information, gas boundary value, additional gas information and inspiration flow rate | — S1101

(b) | Inputting initial value of shot concentration V of mixed vein | — S1102

(c) | Inputting initial value of dead space rate X | — S1103

(d) | Inputting initial value of $CO_2$ partial pressure A2 of pulmonary alveolar gas | — S1104

(e) | Applying input values to operational routine | — S1105

(f) | Calculating estimated value of $O_2$ partial pressure A1 of pulmonary alveolar gas | — S1106

(g) | Obtaining $O_2$ shunt rate Y1 and $CO_2$ shunt rate Y2 based on A2 and A1 | — S1107

S1108

(h) { Whether difference between Y1 and Y2 is within desired range?     No

Yes

Determining pulmonary characteristics related to shot partial pressure A* of pulmonary alveolar gas | — S1109

(i) | Determining plural shot partial pressures (A*)n of pulmonary alveolar gas and pulmonary characteristics | — S1110

S1111

(j) { Whether measured respiratory rate and calculated respiratory rate are within desired range?     No

Yes

Determining specific pulmonary characteristic corresponding to specific shot pressure A** of pulmonary alveolar gas | — S1112

(k) | Calculating cardiac output | — S1113

$$\boxed{\text{End}}$$

FIG. 12

```
                          ( Start )
                              │
(a)    ┌─────────────────────────────────────────────────┐
       │ Inputting additional blood information, gas boundary value, │──── S1201
       │ additional gas information and inspiration flow rate         │
       └─────────────────────────────────────────────────┘
                              │
(b)    ┌─────────────────────────────────────────────────┐
       │ Inputting initial value of shot concentration V of mixed vein │──── S1202
       └─────────────────────────────────────────────────┘
                              │
(c)    ┌─────────────────────────────────────────────────┐
       │        Inputting initial value of shunt rate Y          │──── S1203
       └─────────────────────────────────────────────────┘
                              │
(d)    ┌─────────────────────────────────────────────────┐
       │ Inputting initial value of $CO_2$ partial pressure A2 of │──── S1204
       │ pulmonary alveolar gas                                    │
       └─────────────────────────────────────────────────┘
                              │
(e)    ┌─────────────────────────────────────────────────┐
       │        Applying input values to operational routine      │──── S1205
       └─────────────────────────────────────────────────┘
                              │
(f)    ┌─────────────────────────────────────────────────┐
       │ Calculating estimated value of $O_2$ partial pressure A1 of │──── S1206
       │ pulmonary alveolar gas                                       │
       └─────────────────────────────────────────────────┘
                              │
(g)    ┌─────────────────────────────────────────────────┐
       │ Obtaining $O_2$ dead space rate X1 and $CO_2$ dead space rate X2 │──── S1207
       │ based on A2 and A1                                           │
       └─────────────────────────────────────────────────┘
                              │
                    S1208
(h)         < Whether difference between X1 and X2  >────── No
               is within desired range?
                              │ Yes
       ┌─────────────────────────────────────────────────┐
       │ Determining pulmonary characteristics related to shot partial pressure A* │──── S1209
       │ of pulmonary alveolar gas                                  │
       └─────────────────────────────────────────────────┘
                              │
(i)    ┌─────────────────────────────────────────────────┐
       │ Determining plural shot partial pressures (A*)n of │──── S1210
       │ pulmonary alveolar gas and pulmonary characteristics │
       └─────────────────────────────────────────────────┘
                              │
                    S1211
(j)         < Whether measured respiratory rate    >────── No
               and calculated respiratory rate are
               within desired range?
                              │ Yes
       ┌─────────────────────────────────────────────────┐
       │ Determining specific pulmonary characteristic corresponding │──── S1212
       │ to specific shot pressure A** of pulmonary alveolar gas     │
       └─────────────────────────────────────────────────┘
                              │
(k)    ┌─────────────────────────────────────────────────┐
       │             Calculating cardiac output               │──── S1213
       └─────────────────────────────────────────────────┘
                              │
                          ( End )
```

FIG. 13

Europäisches
Patentamt

European
Patent Office

Office européen
des brevets

**PARTIAL EUROPEAN SEARCH REPORT**        Application Number

which under Rule 63 of the European Patent Convention EP 09 15 4280
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| P,A | EP 1 967 138 A (KOREA INST SCIENCE TECHNOLOGY [KR]) 10 September 2008 (2008-09-10) * the whole document * ----- | 1,10,12, 20 | INV. A61B5/029 A61B5/083 |
| A | WO 2006/119546 A (PEYTON PHILIP JOHN [AU]) 16 November 2006 (2006-11-16) * the whole document * ----- | 1,10,12, 20 | |
| A | US 6 402 697 B1 (CALKINS JERRY M [US] ET AL) 11 June 2002 (2002-06-11) * the whole document * ----- | 1,10,12, 20 | |
| A | WO 2004/073482 A (FISHER JOSEPH [CA]; PREISS DAVID [CA]; AZAMI TAKAFUMI [CA]; VESELY ALE) 2 September 2004 (2004-09-02) * the whole document * ----- | 1,10,12, 20 | |
| A | WO 00/13581 A (NTC TECHNOLOGY INC [US]) 16 March 2000 (2000-03-16) * the whole document * ----- | 1,10,12, 20 | |

TECHNICAL FIELDS
SEARCHED      (IPC)

A61B

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 17 April 2009 | Dydenko, Igor |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
    .........................................................................
& : member of the same patent family, corresponding
    document

EPO FORM 1503 03.82 (P04E07)

Europäisches
Patentamt

European
Patent Office

Office européen
des brevets

**INCOMPLETE SEARCH
SHEET C**

Application Number

EP 09 15 4280

Claim(s) searched incompletely:
1-22

Claim(s) not searched:
-

Reason for the limitation of the search:

The method claims 1-19 appear to be a sparse aggregation of parameters,
many of which are unclear or undefined, of "calculating" and
"determining" steps using undefined expressions and relating to results
to be achieved. It is therefore impossible to determine any precise
technical features of the method which would allow to understand the
matter for which protection is sought and to compare the claimed matter
to the prior art.

Independent claim 20 merely defines a display device suitable for
displaying pulmonary characteristics; it is not clear whether the
reference to the way of determining the parameter is meant to impose any
limitation on the claimed subject-matter. Further, as specified above,
the method claims referred to from claim 20 are not clear.

Search was carried out in so far as the claims could be interpreted in
the light of the description and the figures.

**ANNEX TO THE EUROPEAN SEARCH REPORT
ON EUROPEAN PATENT APPLICATION NO.**

EP 09 15 4280

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2009

| Patent document cited in search report | | Publication date | Patent family member(s) | | Publication date |
|---|---|---|---|---|---|
| EP 1967138 | A | 10-09-2008 | JP 2008212686 A<br>KR 20080081388 A<br>US 2008221460 A1 | | 18-09-2008<br>10-09-2008<br>11-09-2008 |
| WO 2006119546 | A | 16-11-2006 | NONE | | |
| US 6402697 | B1 | 11-06-2002 | NONE | | |
| WO 2004073482 | A | 02-09-2004 | CA 2522623 A1<br>EP 1601281 A2<br>JP 2006517813 T<br>US 2007062531 A1 | | 02-09-2004<br>07-12-2005<br>03-08-2006<br>22-03-2007 |
| WO 0013581 | A | 16-03-2000 | US 6042550 A<br>US 6241681 B1<br>US 6258038 B1 | | 28-03-2000<br>05-06-2001<br>10-07-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20080020254
- KR 19870002027
- KR 1019990000417
- KR 199922493